(19) Europäisches Patentamt / European Patent Office / Office européen des brevets

(11) EP 2 603 488 B1

(12) EUROPÄISCHE PATENTSCHRIFT

(45) Veröffentlichungstag und Bekanntmachung des Hinweises auf die Patenterteilung:
**26.04.2017 Patentblatt 2017/17**

(21) Anmeldenummer: **11741249.4**

(22) Anmeldetag: **10.08.2011**

(51) Int Cl.:
***C07C 323/12*** (2006.01) ***C09D 175/04*** (2006.01)
***G03C 1/73*** (2006.01) ***G03F 7/00*** (2006.01)
***G03F 7/004*** (2006.01) ***G03F 7/027*** (2006.01)
***G03H 1/02*** (2006.01) ***G03H 1/30*** (2006.01)
***G11B 7/24044*** (2013.01) ***G11B 7/245*** (2006.01)

(86) Internationale Anmeldenummer:
**PCT/EP2011/063785**

(87) Internationale Veröffentlichungsnummer:
**WO 2012/020061 (16.02.2012 Gazette 2012/07)**

(54) **DIFUNKTIONELLE (METH)ACRYLAT-SCHREIBMONOMERE**
DIFUNCTIONAL (METH)ACRYLATE WRITING MONOMERS
MONOMÈRES D'ENREGISTREMENT DIFONCTIONNELS À BASE DE (MÉTH)ACRYLATE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.08.2010 EP 10172536**

(43) Veröffentlichungstag der Anmeldung:
**19.06.2013 Patentblatt 2013/25**

(73) Patentinhaber: **Covestro Deutschland AG**
**51373 Leverkusen (DE)**

(72) Erfinder:
- **FÄCKE, Thomas**
**51375 Leverkusen (DE)**
- **BRUDER, Friedrich-Karl**
**47802 Krefeld (DE)**
- **RÖLLE, Thomas**
**51381 Leverkusen (DE)**
- **WEISER, Marc-Stephan**
**51377 Leverkusen (DE)**
- **HÖNEL, Dennis**
**53909 Zülpich-Wichterich (DE)**
- **BERNETH, Horst**
**51373 Leverkusen (DE)**

(74) Vertreter: **Levpat**
**c/o Covestro AG**
**Gebäude 4825**
**51365 Leverkusen (DE)**

(56) Entgegenhaltungen:
**EP-A1- 1 627 867**

- **DATABASE WPI Week 200876 Thomson Scientific, London, GB; AN 2008-N01620 XP002609997, -& WO 2008/120573 A1 (NIPPON KAYAKU KK) 9. Oktober 2008 (2008-10-09)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 603 488 B1

## Beschreibung

Die Erfindung betrifft eine Verbindung, die insbesondere als Schreibmonomer in Photopolymer-Formulierungen verwendet werden kann. Weiterhin sind eine Photopolymer-Formulierung umfassend wenigstens eine Polyol-Komponente, eine Polyisocyanat-Komponente, ein Schreibmonomer und einen Photoinitiator sowie die Verwendung der Photopolymer-Formulierung zur Herstellung holographischer Medien ebenfalls Gegenstände der Erfindung.

Für die Verwendungen von Photopolymer-Formulierungen spielt die durch die holographische Belichtung im Photopolymer erzeugte Brechungsindexkontrast $\Delta n$ die entscheidende Rolle. Bei der holographischen Belichtung wird das Interferenzfeld aus Signal- und Referenzlichtstrahl (im einfachsten Fall das zweier ebener Wellen) durch die lokale Photopolymerisation von z.B. hochbrechenden Acrylate an Orten hoher Intensität im Interferenzfeld in ein Brechungsindexgitter abgebildet. Das Brechungsindexgitter im Photopolymeren (das Hologramm) enthält alle Information des Signallichtstrahls. Durch Beleuchtung des Hologramms nur mit dem Referenzlichtstrahl kann dann das Signal wieder rekonstruiert werden. Die Stärke des so rekonstruierten Signals im Verhältnis zur Stärke des eingestrahlten Referenzlichts wird Beugungseffizienz, im folgenden DE wie Diffraction Efficiency, genannt.

Im einfachsten Fall eines Hologramms, das aus der Überlagerung zweier ebener Wellen entsteht, ergibt sich die DE aus dem Quotienten der Intensität des bei der Rekonstruktion abgebeugten Lichtes und der Summe der Intensitäten aus eingestrahltem Referenzlicht und abgebeugtem Licht. Je höher die DE desto effizienter ist ein Hologramm in Bezug auf die Lichtmenge des Referenzlichtes, die notwendig ist, um das Signal mit einer festen Helligkeit sichtbar zu machen.

Hochbrechende Acrylate sind in der Lage, Brechungsindexgitter mit hoher Amplitude zwischen Bereichen mit niedrigem Brechungsindex und Bereichen mit hohem Brechungsindex zu erzeugen und damit in Photopolymer-Formulierungen Hologramme mit hohem DE und hohem $\Delta n$ zu ermöglichen. Dabei ist zu beachten, dass DE vom Produkt aus $\Delta n$ und der Photopolymerschichtdicke d abhängt. Die Breite des Winkelbereiches bei dem das Hologramm z.B. bei monochromatischer Beleuchtung sichtbar (rekonstruiert) wird, hängt nur von der Schichtdicke dab.

Bei Beleuchtung des Hologramms mit z.B. weißem Licht hängt die Breite des spektralen Bereiches, der zur Rekonstruktion des Hologramms beitragen kann, ebenfalls nur von der Schichtdicke d ab. Dabei gilt je kleiner d desto größer die jeweiligen Akzeptanzbreiten. Will man daher helle und leicht sichtbare Hologramme herstellen, ist ein hohes $\Delta n$ und eine geringe Dicke d anzustreben und zwar so, dass DE möglichst groß wird. Das heißt je höher $\Delta n$ wird, wird, desto mehr Freiraum zur Gestaltung der Schichtdicke d für helle Hologramme erreicht man ohne Verlust an DE. Daher kommt der Optimierung von $\Delta n$ bei der Optimierung von Photopolymer-Formulierungen eine herausragenden Bedeutung zu (P. Hariharan, Optical Holography, 2nd Edition, Cambridge University Press, 1996).

Aus der WO 2008/125229 A1 sind Photopolymer-Formulierungen bekannt die hochmolekulare, mono- und difunktionelle Schreibmonomere enthalten. In Medien aus diesen Formulierungen können Reflexions-Hologramme geschrieben werden, die sich beispielsweise gut für die Datenspeicherung eignen. Allerdings treten bei der Herstellung und Verarbeitung der Formulierungen Probleme auf: So weisen die enthaltenen Schreibmonomere eine große Viskosität bzw. hohe $T_G$-Werte ($T_G$ = Glasübergangstemperatur) auf. Dies bedingt, dass eine gleichmäßige Verteilung der Schreibmonomere in der Photopolymer-Formulierung und einem daraus hergestellten Medium schwierig zu realisieren ist. Außerdem kann es bei der Verwendung der bekannten Formulierungen in der Polymermatrix zur Bildung von Schreibmonomer-Agglomeraten kommen, was die Qualität der Medien bzw. der darin eingeschriebenen Hologramme erheblich beeinträchtigt. In derartigen Fällen werden die holographischen Materialien trüb.

In der WO 2008/125199 sind wiederum trifunktionelle Urethanacrylat-Schreibmonomere und diese enthaltende Photopolymer-Formulierungen beschrieben. Besonders hoher Brechungsindexkontrast kann hiermit erzielt werden, wenn die Formulierungen zusätzlich spezielle Fluorurethane enthalten. Derartige Fluorurethane bzw. deren Verwendung in Photopolymer-Formulierungen sind beispielsweise in der noch nicht veröffentlichten europäischen Anmeldung mit der Anmeldenummer sind EP 09013770.4 beschrieben.

Grundsätzlich steigt bei diesen Formulierungen der Brechungsindexkontrast mit dem Gehalt an Fluorurethan an. Allerdings kommt es mit zunehmendem Gehalt an Fluorurethan zu Beeinträchtigungen der optischen Qualität durch Trübungen.

In der JP 2008 247755 A sind Di(meth)acrylate beschrieben, die als Schreibmonomere für die Erzeugung von Hologrammen verwendet werden können.

Eine besondere Form von Hologrammen sind Transmissions-Hologramme, die sich dadurch auszeichnen, dass beim Erzeugen der Hologramme der Referenz- und der Objektstrahl das holografische Medium von derselben Seite bestrahlen. Transmissions-Hologramme finden vielfältige Anwendungen. Insbesondere ist hier die Lichtlenkung als diffraktives optisches Element zu nennen. Derartige optische Elemente können in anspruchsvollen Anwendungen wie der Spektroskopie oder der Astronomie zum Einsatz kommen. Ebenso eignen sie sich zur Verwendung in elektronischen 3D- Displays.

Bedingt durch die Geometrie der zur Interferenz gebrachten Objekt- und Signalstrahlen ist der Gitterabstand in Transmissions-Hologrammen im Vergleich zu Reflexions-Bedingt durch die Geometrie der zur Interferenz gebrachten

Objekt- und Signalstrahlen ist der Gitterabstand in Transmissions-Hologrammen im Vergleich zu Reflexions-Hologrammen groß. Je nach Wellenlänge kann er zwischen 500-1000 nm betragen. Da der Mechanismus der Hologrammbildung in den Photopolymer-Formulierungen auf der Diffusion der Schreibmonomere beruht, ist es schwierig Schreibmonomere zu entwickeln, die bei dem für Transmissions-Hologramme üblichen großen Gitterabstand hinreichend weit diffundieren können. Dies ist jedoch Voraussetzung, um einen hoher Brechungsindexkontrast (An) ermöglichen zu können. Die aus dem Bereich der Reflexionshologramme bekannten Photopolymere sind hierfür häufig nicht geeignet, bzw. führen nicht zu einem ausreichenden hohen Brechungsindexkontrast.

Aufgabe der vorliegenden Erfindung war es daher, eine Verbindung bereitzustellen, die sich als Schreibmonomer zur Herstellung von holographischen Medien für Transmissionshologramme mit hohem Brechungsindexkontrast (An) eignen. Darüber hinaus sollte sie bevorzugt auch nicht zu einer Beeinträchtigung der optischen Qualität der holographischen Medien etwa durch Trübungen beitragen.

Diese Aufgabe ist durch eine Verbindung der Formel (I)

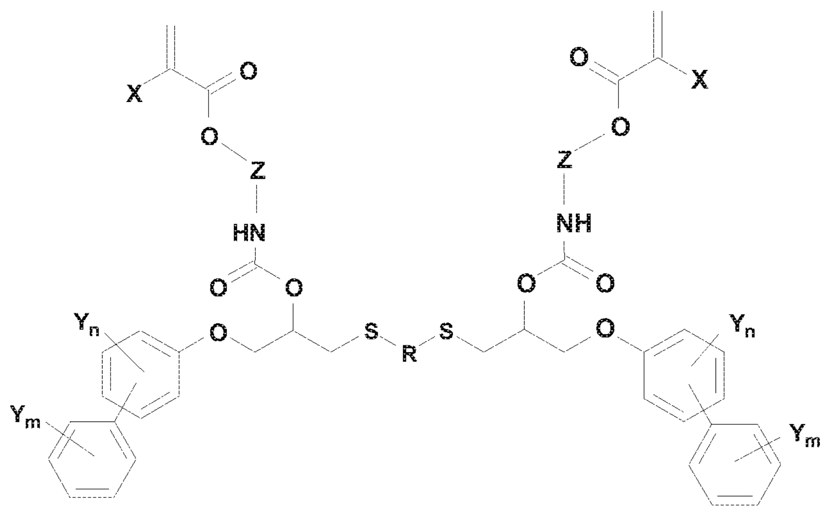

Formel (I)

gelöst
in der

X $CH_3$ oder Wasserstoff,

Z ein linearer oder verzweigter C2 bis C4-Alkylrest,

R ein linearer oder verzweigter, gegebenenfalls mit Heteroatomen substituierter aliphatischer, aromatischer oder araliphatischer Rest, oder ein linearer oder verzweigter, gegebenenfalls mit Heteroatomen substituierter aliphatischer, aromatischer oder araliphatischer Ether (-O-), Thioether (-S-), Ester (-O-CO) oder Urethan (-NH-(C=O)-O-),

Y jeweils unabhängig voneinander Wasserstoff, Methyl, Ethyl, Propyl, n-Butyl, tert.-Butyl, Chlor, Brom, Iod, Methylthio, Phenyl oder Phenylthio,

n 0 bis 4 und

m 0 bis 5

sind

So wurde gefunden, dass mit Hilfe der erfindungemäßen Verbindungen Photopolymer-Formulierungen erhältlich sind, aus denen wiederum holographische Medien insbesondere zur Aufnahme von Transmissions-Hologrammen hergestellt werden können. Diese Medien weisen nicht nur eine hohe optische Qualität auf, sondern liefern darüber hinaus insbesondere bei Transmissions-Hologrammen auch einen hohen Brechungsindexkontrast ($\Delta$n).

Aus der EP 1 627 867 A1 sind zwar strukturell zur Formel (I) ähnliche Verbindungen bekannt, bei deren Verwendung als Schreibmonomere zur Herstellung von Transmissionshologrammen kann jedoch nicht der erfindungsgemäß zu erzielende hohe Brechungsindexkontrast realisiert werden. Dies ist durch ein Vergleichsbeispiel im experimentellen Teil der Anmeldung belegt.

Gemäß einer ersten bevorzugten Ausführungsform ist vorgesehen, dass R ein linear oder verzweigte aliphatischer, aromatischer oder araliphatischer Rest mit 2 bis 22 Kohlenstoff-Atomen ist, der bevorzugt mit einem oder mehreren Sauerstoff-, Stickstoff- und/oder Schwefel-Atomen substituiert ist. Weiter bevorzugt ist, wenn R 2 bis 16 Kohlenstoff-, 0 bis 4 Sauerstoff-, 0 bis 1 Stickstoff- und 0 bis 1 Schwefel-Atome aufweist. In diesen Fällen kann ein besonders hoher Brechungsindexkontrast realisiert werden.

Möglich ist auch, dass R wenigstens eine funktionelle Gruppe ausgewählt aus der Gruppe Ether (-O-), Thioether (-S-), Ester (-O-CO), Urethan (-NH-(C=O)-O-) umfasst. Es kann sich bei R in diesem Fall also insbesondere um lineare oder verzweigte, gegebenenfalls mit Heteroatomen substituierte aliphatische, aromatische oder araliphatische Ether, Thioether, Ester oder Urethane handeln, wobei diese Verbindungen wiederum bevorzugt aliphatischer Natur sein können.

Ganz besonders bevorzugt ist, wenn R $(CH_2)_1$ mit 1=2 bis 10, $(CH_2CH_2\text{-}O)_m\text{-}CH_2\text{-}CH_2$ mit m = 1 oder 2, $CH(CH_3)\text{-}CH(CH_3)$, $CH_2\text{-}CO\text{-}OCH_2\text{-}CH_2\text{-}O\text{-}CO\text{-}CH_2$, Phenylen-S-Phenylen und/oder $CH_2\text{-}CH(CH_2\text{-}O\text{-}CO\text{-}NH\text{-}$Phenylen-S-Phenyl) ist.

Bevorzugt sind auch Verbindungen der Formel (I), bei denen X Wasserstoff ist.

Die Substituenten Y können jeweils unabhängig voneinander H, Methyl, Phenyl, Methyllthio oder Phenylthio und bevorzugt Wasserstoff sein.

Gemäß einer weiteren bevorzugten Ausführungsform können bei der Verbindung der Formel (I) $n \geq 1$ und $\leq 4$ und/oder $m \geq 1$ und $\leq 5$ sein. Weiter bevorzugt kann $m + n < 4$ sein.

Vorteilhaft ist weiter, wenn die Gruppen Z jeweils ein $(CH_2)_i$-Rest mit $i \geq 2$ und $\leq 4$ oder ein-CH2-CH($CH_3$) -Rest sind. Ganz besonders bevorzugt sind Z $\text{-}CH_2\text{-}CH_2$-Reste.

Die erfindungsgemäßen Di(meth)acrylate können in einem zwei stufigen Verfahren hergestellt werden. Dabei wird zunächst in einem ersten Schritt ein monofunktioneller Biphenylglycidether mit einem difunktionellem Thiol umgesetzt. Die Addition erfolgt streng an der sterisch weniger anspruchsvollen Seite des Oxirans, so dass immer sekundäre Alkohole gebildet werden. Anschließend wird der entstandene Alkohol zweimal an 2-Isocyanatoalkyl(meth)acrylat zum erfindungsgemäßen Di(meth)acrylat addiert. Dieses Verfahren ist beispielhaft in dem folgenden zweistufigem Reaktionsschema dargestellt:

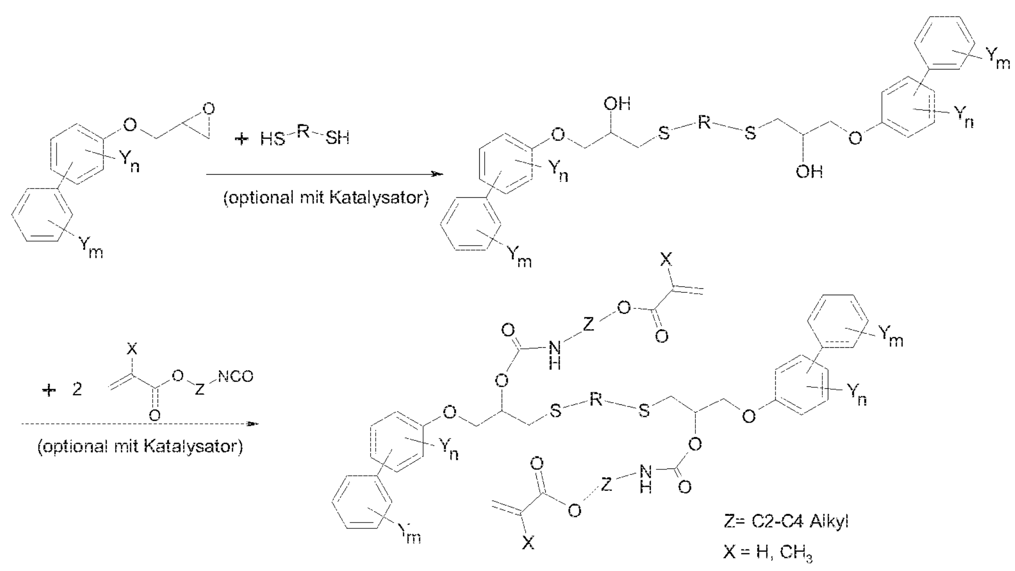


Für die Herstellung der erfindungsgemäßen Verbindungen können monofunktionelle Biphenylglycidether eingesetzt werden. Diese können aus den entsprechenden Phenylphenolen z.B. durch Umsetzung mit Epichlorhydrin oder Epibromhydrin in Gegenwart von Basen zum Neutralisieren der entstehenden Chlor- bzw. Bromwasserstoffsäure hergestellt werden. Als Base eignet sich hier z.B. Kaliumcarbonat.

Geeignete Phenylphenole sind ortho-Phenylphenol, meta-Phenylphenol und para-Phenylphenol. Es können auch substituiertes ortho-Phenylphenol bzw. meta-Phenylphenol bzw. para-Phenylphenol eingesetzt werden. Die Phenylphenole können auch mehrfach substituiert sein, wobei gleiche sowie verschiedene Substituenten an einem der beiden Phenylringe oder an beiden Ringen des Phenylphenols möglich sind. Mögliche Substituenten Y sind kurzkettige Alkylreste wie Methyl, Ethyl, Propyl, n-Butyl, t.-Butyl, und/oder Halogenatome wie Chlor, Brom, Iod, sowie Thioether wie

Methylthio, Phenylthio, und/oder aromatische Substituenten wie Phenyl.

Bevorzugte Substituenten Y sind Methyl, Phenyl, Methylthio und/oder Phenylthio. Besonders bevorzugt ist dabei, wenn nicht mehr als drei gleiche oder verschiedene Substituenten auf beide Ringe verteilt sind(m+n <4).

Weiter bevorzugt sind mono- und nicht substituierte Phenylphenole mit Y= H, Methyl, Phenyl, Methylthio und Phenylthio. Ganz besonders bevorzugt sind ortho-Phenylphenol und meta-Phenylphenol.

Für die Herstellung der erfindungsgemäßen Di(meth)acrylate können aliphatische, aromatische und/oder araliphatische sowohl lineare und/oder verzweigte, difunktionelle Thiole mit 1-40 Kohlenstoff-, sowie gegebenenfalls zusätzlich Sauerstoff-, Stickstoff-, Phosphor-, Schwefel-, Chlor-, Brom- und Iod-Atomen verwendet werden.

Bevorzugte difunktionelle Thiole enthalten in der Summe 4-22 Kohlenstoff, Sauerstoff, Stick-stoff oder Schwefel-Atome, besonders bevorzugte enthalten 2-16 Kohlenstoff, 0-4 Sauerstoff, 0-1 Stickstoffatom und 0-3 Schwefel-Atome.

Generell bevorzugt sind difunktionelle Thiole, die eine oder mehrere Ether (-O-), Thioether (-S-), Ester (-O-CO), Thioester (-O-CS-), Dithioester (-S-CS-), Urethan (NH-CO), Thiourethane (NH-CS), ortho-, meta- und para Phenylen (-Phenyl-), Methylthio (MeS-), Phenylthio (PhS-), Phenyl (Ph-), Methyl ($CH_3$-) und/oder Napthyl ($C_{10}H_7$-) Gruppen enthalten.

Besonders bevorzugte Dithiole sind solche Verbidnungen, die eine oder mehrere Ether (-O-), Thioether (-S-), Ester (-O-CO), Urethan (NH-CO), ortho-, meta- und para Phenylen (-Phenyl-), Methylthio (MeS-) und/oder Phenylthio (PhS-) Gruppen enthalten.

Es können aliphatische unverzweigte Dithiole wie 1,2-Ethandithiol, 1,3-Fropandithiol, 1,4-Butandithiol, 1,5-Pentandithiol, 1,6-Hexandithiol, 1,7-Heptandithiol, 1,8-Oktandithiol, 1,9-Nonandithiol, 1,10-Decandithiol, 1,11-Undecandithiol,1,12-Dodecandithiol, 1,16-Hexadecandithiol, 1,18-Octadecandithiol, als auch verzweigte Dithiole wie z. B. 1,2-Propandithiol, 1,2-Butandithiol, 2,3-Butandithiol, 1,3-Butandithiol, die isomeren Pentandithiole (z.B. 2,3-Pentanditiol), Hexandithiole (z.B: 3,4- Hexandithiole), Heptanditiol, Oktandithiole (z.B. 2-Ethylhexandithiol-1,3), Nonandithiole, Decandithiole, Undecandithiole, Dodecandithiole und Hexadodecandithiol, Octadecandithiole sowie cycloaliphatische Dithiole wie z.B. 2-Methyl-4-(1-sulfanylpropan-2-yl)cyclohexanethiol zum Einsatz kommen.

Ether- und Thioether haltige Dithiole, wie z.B. 2,2'-Oxydiethanthiol, 2,2'-Sulfanediyldiethanthiol (DMDES), 1,2-Bis(2-Mercaptoethoxy)ethan, 2,2'-[Oxybis(ethan-2,1-diyloxy)]diethanthiol, wie auch Estergruppen haltige Dithiole wie z.B. Ethylenglycoldimercaptoacetat, Ethylenglycoldimercaptoproprionat, Propylenglycoldimercaptoacetat, Propylenglycoldimercaptoproprionat, Butylenglycoldimercaptoacetat, Butylenglycoldimercaptoproprionat, Hexylenglycoldimercaptoacetat, Hexylenglycoldimercaptoproprionat wie auch Ether- und Esterhaltige Dithiole wie z.B. Diethylenglycoldimercaptoacetat, Diethylenglycoldimercaptoproprionat können ebenfalls verwendet werden.

Weiterhin sind auch aromatische Dithiole wie z.B. 1,2-Benzendithiol, 1,3-Benzendithiol, 1,4-Benzendithiol, 4,4'-Sulfanediyldibenzolthiol (4,4'-Thiobisbenzenthiol) und araliphatische Dithiole wie z.B. 1,4-Benzendimethanthiol, 1,3-Benzendimethanthiol, 1,2-Benzendimethanthiol und 2-(Mercaptomethyl)phenylmethanthiol, 3-(Mercaptomethyl)phenyl methanthiol und/oder 4-(Mercaptomethyl)phenyl methanethiol geeignet.

Schließlich lassen sich auch Alkoholgruppen tragendende Dithiole wie z.B. 2,3-Disulfanylpropan-1-ol und/oder 1,4-Disulfanylbutan-2,3-diol verwenden. Bei diesen können dabei in der zweiten Stufe die Umsetzung nicht nur der durch die Thiol-Oxiran Addition entstehenden Alkohole urethanisiert werden, sondern auch die schon vorliegenden Alkoholgruppen der Alkoholgruppen tragendenden Dithiole.

Dabei ist es möglich, die Alkoholgruppe vor der Addition der Thiolgruppen an das Oxiran zu urethanisieren. Alternative kann die Urethanisierung nach der Addition erfolgen. Dabei spielt es eine besondere Rolle, die unterschiedliche Reaktivität von primären zu sekundären oder tertiären Alkohole auszunutzen zu können. So kann z.B. die primäre Alkoholgruppe des 2,3-Disulfanylpropan-1-ol vor oder nach der Thiol-Oxiran Reaktion zunächst selektiv urethanisiert werden und dann bei höheren Temperaturen bzw. bei höherer Katalysatormenge die se-kundären Alkoholgruppen aus der Thiol-Oxiran Reaktion umgesetzt werden. Durch dieses Vorgehen ist es möglich, entweder alle Alkoholgruppen mit einem Isocyanato(meth)acrylat oder die primäre Alkoholgruppe mit einem Monoisocyanat und die sekundären Alkoholgruppen mit Isocyanato(meth)acrylat umzusetzen.

Geeignete Monoisocyanate für eine derartige Urethanisierung sind Phenylisocyanat, 2-, 3- und 4-Tolylisocyanat, die isomeren 2-, 3-, und 4-Biphenylisocyanat, 3,4-Dichlorphenylisocyanat, die isomeren 2-, und 3-Napthylisocyanat, die isomeren 2-, 3-, und 4-Methylthiophenylisocyanat und die isomeren 2-, 3-, und 4-Phenythiophenylisocyanat. Bevorzugt sind Methylthiophenylisocyanat und Phenylthiophenylisocyanat.

Die Thiol-Oxiran Reaktion kann ohne Katalysator durchgeführt werden, was aber in einigen Fällen zu sehr langen Reaktionszeiten bzw. zu niedrigen Umsätze führt. Wenn kein Katalysator verwendet wird, sind häufig hohe Temperaturen nötig, die dann die Farbzahl der Produkte unerwünscht erhöhen können.

Daher ist es von Vorteil, einen Katalysator zuzusetzen, typischerweise in der Menge von 0,02-1 Gew.-%. Dieser kann von Anfang an in der Reaktionsmischung vorhanden sein oder auch erst während der Reaktion zugesetzt werden.

Es können verschiedene Substanzklassen als Katalysatoren verwendet werden: Beispiele sind Broenstedsäuren wie Phosphorsäure, phosphorige Säure, Schwefelsäure; Lewissäuren wie Zincacetate, Zinccetylacetonate, Titan-IV-methoxid, Tetrakis(dimethylamino)zirkonium, Lewisbasen wie 2-Methylimidazole, Dimethylaminopyridin, Boran-Pyridin-Komplex, Tris(dimethylamino)boran, Triphenylphosphin, Tris(o-tolyl)phosphin, Choline chloride, Tris(4-dimethylenaminophenyl)phosphin, Tris(4-methoxyphenyl)phosphin, 1,4,5,6-Tetrahydropyrimidin, Diazabicycloundecan (DABCO) und andere Amine, und Ammonium oder Phosphoniumsalze, wie z.B. Tetraethylammoniumtrifluoracetat, Tetrabutylphosphoniumbromid, Benzyltrimethylammonium bromid, Benzyltrimethylammonium chlorid, 1-Butyl-1-methylpyrrolidinium Bromide, 1-Butyl-1-methylpyrrolidinium Chloride, Tetrabutylphosphoniumchlorid und Tetrabutylphosphoniumbromid, Tetraphenylphosphoniumchlorid und Tetraphenylphosphoniumbromid, Triphenylbutylphosphoniumchlorid und Triphenylbutylphosphoniumbromid, Triphenylethylphosphoniumchlorid und Triphenylethylphosphoniumbromid als auch Tetrakis(dimethylamino)silan.

Auch Imidazole wie 1,2-Dimethyl-3-propylimidazolium Iodid, 1,3-Dimethylimidazolium Chlorid, 1-Butyl-2,3-dimethylimidazolium Chlorid, 1-Butyl-3-methylimidazolium Bromid, 1-Butyl-3-methylimidazolium Chlorid, 1-Butyl-3-methylimidazolium Iodid, 1-Ethyl-3-methylimidazolium Bromid, 1-Ethyl-3-methylimidazolium Chlorid, 1-Ethyl-3-methylimidazolium Iodid, 1-Ethyl-3-methylimidazolium Iodid, 1-Hexyl-3-methylimidazolium Bromid, 1-Hexyl-3-methylimidazolium Chlorid, 1-Methyl-3-n-octylimidazolium Bromid, 1-Methyl-3-n-octylimidazolium Chlorid, 1-Methyl-3-propylimidazolium Iodid können verwendet werden.

Weiterhin sind Pyridine wie z.B: 1-Butyl-3-methylpyridinium Bromid, 1-Butyl-3-methylpyridinium Chlorid, 1-Butyl-4-methylpyridinium Bromid, 1-Butyl-4-methylpyridinium Chlorid, 1-Butylpyridinium Bromid, 1-Butylpyridinium Chlorid, 1-Ethylpyridinium Bromid, 1-Ethylpyridinium Chlorid geeignet.

Bevorzugt sind Triphenylphosphin und 1-Butyl-3Methylimidazoliumbromid.

Die Reaktionsführung der Thioladdition an das Oxiran kann, je nach Reaktivität bei Temperaturen zwischen 20 und 100 °C und typischerweise bei 60 °C, durch Vorlage des Oxirans, und des Katalysators und anschließendes langsamen Zutropfens des Thiols erfolgen. Das Thiol kann ebenfalls vorgelegt und das Oxiran dann zugetropft werden.

Um eine zügige und vollständige Umsetzung zu erzielen, wird bei Reaktionstemperatur bis maximal 130 °C gearbeitet, wobei auch höhere Temperaturen möglich sind, dann aber Nebenreaktionen und unerwünschte Farbbildung beobachtet werden. Die Reaktion kann unter Sauerstoffabschluß durchgeführt werden. Dies ist aber in der Regel nicht nötig. Typischerweise werden nach der leicht bis recht heftig exothermen Reaktion während des Zutropfens anschließend Temperaturen von 70 bis 90 °C eingestellt, um die Reaktion abzuschließen.

Ein leichter Oxiranüberschuß (maximal 10 mol%) kann von Vorteil sein, um den störenden Geruch nicht umgesetzten Thiols zu vermeiden. Insbesondere bei den niedermolekularen leichter flüchtigen Dithiolen ist dies von Vorteil.

Das Reaktionsende kann mittels $^1$H-NMR Spektroskopie festgestellt werden (das Oxiran liefert charakteristische Resonanzen im $^1$H-NMR (400 MHz, CDCl3): $\delta$=2,6 (dd), 2,8 (dd), 3,2 (m)).

Es können zusätzlich Lösungsmittel zugesetzt werden, um die Viskosität der Reaktion zu kontrollieren. Dies kann insbesondere bei aromatischen und araliphatischen Dithiolen vorteilhaft sein. Aliphatische Dithiole können dagegen in der Regel ohne Lösungsmittel umgesetzt werden.

In dem zweiten Reaktionsschritt werden die Alkohole urethanisiert.

Die Urethanisierung erfolgt typischerweise bei 20-180 °C, bevorzugt bei 40-120 °C und besonders bevorzugt bei 50-100 °C. Dabei kann der Alkohol als Produkt der ersten Stufe vorgelegt, gegebenfalls mit einem Katalysator versetzt und dann das Isocyanat zugetropft werden.

Die Reaktion ist beendet, wenn der NCO-Gehalt unter 1%, bevorzugt unter 0,1 Gewichts-% gefallen ist. Der NCO-Gehalt kann dabei mittels IR-Spektroskopie oder durch Titration bestimmt werden.

Es ist ebenfalls möglich, zunächst das Isocyanat vorzulegen und dann den Alkohol zuzutropfen. Die bevorzugte Art der Zugabe wird im konkreten Fall von der Handhabung und damit von der Viskosität der Ausgangstoffe beeinflusst.

Als Katalysatoren können für die Urethanisierung Amine sowie Metallverbindungen der Metalle Zinn, Zink, Eisen, Bismuth, Molybdän, Kobalt, Calzium, Magnesium und Zirkonium verwendet werden. Bevorzugt sind Zinnoctoat, Zinkoktoat, Dibutylzinndilaurat, Dimethylzinndicarboxylat, Eisen(III)-acelylacetonat, Eisen(II)-chlorid, Zinkchlorid, Tetraalkyl-ammoniumhydroxide, Alkalihydroxide, Alkalialkoholate, Alkalisalze von langkettigen Fettsäuren mit 10 bis 20 Kohlenstoffatomen und gegebenenfalls seitenständigen OH-Gruppen, Bleioctoat oder tertiäre Amine wie Triethylamin, Tributylamin, Dimethylbenzylamin, Dicyclohexylmethylamin, Dimethyl-cyclohexylamin, N,N,N',N'-Tetramethyl-diamino-diethylether, Bis-(dimethylamino-propyl)-harnstoff, N-Methyl- bzw. N-Ethylmorpholin, N,N'-Dimorpholinodiethylether (DMDEE), N-Cyclohexylmorpholin, N,N,N',N'-Tetramethylethylendiamin, N,N,N',N'-Tetramethyl-butan-diamin, N,N,N',N'-Tetramethylhexandiamin-1,6, Pentamethyldiethylentriamin, Dimethylpiperazin, N-Dimethylaminoethylpiperidin, 1,2-Dimethylimidazol, N-Hydroxypropylimidazol, 1-Azabicyclo-(2,2,0)-octan, 1,4-Diazabicyclo-(2,2,2)-octan (Dabco) oder Alkanolaminverbindungen, wie Triethanolamin, Triisopropanolamin, N-Methyl- und N-Ethyl-diethanolamin, Dimethyl-amino-ethanol, 2-(N,N-Dimethylaminoethoxy)ethanol oder N-Tris-(dialkylaminoalkyl)hexa-hydro-triazine, z.B. N,N',N-Tris-(dimethylaminopropyl)-s-hexahydrotriazin, Diazabicyclononan, Diazabicycloundecan, 1,1,3,3-Tetramethylguanidin, 1,3,4,6,7,8-Hexahydro-1-methyl-2H-pyrimido(1,2-a)pyrimidin.

Besonders bevorzugte Katalysatoren sind Dibutylzinndilaurat, Dimethylzinndicarboxylat, Eisen(III)acetylacetonat, 1,4-Diazabicyclo-[2.2.2]-octan, Diazabicyclononan, Diazabicycloundecan, 1,1,3,3-Tetramethylguanidin, 1,3,4,6,7,8-Hexahydro-1-methyl-2H-pyrimido(1,2-a)-pyrimidin.

Während der Urethanisierung wird üblicherweise Luft durchgeleitet, um eine unerwünschte Polymerisation zu vermeiden. Dabei muss darauf geachtet werden, dass ausreichend Phenole wie z.B. p-Methoxyphenol oder Jonol (2,6) anwesend sind, wobei Mengen zwischen 0,001-0,1 Gewichtsprozent vorteilhaft sind. Als Radikalstabilisatoren geeignet sind Inhibitoren und Antioxidantien wie sie in "Methoden der organischen Chemie" (Houben-Weyl), 4. Auflage, Band XIV/1, S. 433ff, Georg Thieme Verlag, Stuttgart 1961, beschrieben sind. Geeignete Stoftklassen sind beispielsweise Phenole wie z.B. 2,6-Di-tert-butyl-4-methylphenol, Kresole, Hydrochinone, Benzylalkohole wie z.B. Benzhydrol, ggf. auch Chinone wie z. B. 2,5-Di-tert.-Butylchinon, ggf. auch aromatische Amine wie Diisopropylamin oder Phenothiazin. Bevorzugte Radikalstabilisatoren sind 2,6-Di-tert.-butyl-4-methylphenol, Phenothiazin und Benzhydrol.

Die erfindungsgemäßen Verbindungen wird durch Umsetzung der beiden sekundären Alkoholgruppen, die durch die Addition des Dithiols an das Monooxiran gebildet worden, mit einer Acrylat-haltigen Isocyanat-Komponente erhalten, wobei als Isocyanat-Komponenten insbesondere Isocyanatoethylacrylat oder Isocyanatoethylmethacrylat verwendet werden können. Eine Mischung aus beiden Komponenten kann ebenfalls eingesetzt werden.

Verwendet man ein Dithiol, dass zusätzlich noch Alkoholgruppen trägt, können diese ebenfalls mit einer Acrylathaltiger Isocyanatkomponente umgesetzt werden, wodurch höher funktionelle Acrlyate erhalten werden können. Es ist ebenfalls möglich, eine solche zusätzliche Alkoholgruppe selektiv, wie oben beschrieben, mit Monoisocyanaten ohne Acrylatfunktion umzusetzten. In diesem Fall wird eine Urethangruppe innerhalb der Gruppe R eingefügt.

Ein weiterer Gegenstand der Erfindung ist die Verwendung einer Verbindung gemäß Formel (I) als Schreibmonomer in einer Photopolymer-Formulierung.

Gegenstand der Erfindung ist weiterhin eine Photopolymer-Formulierung umfassend wenigstens eine Polyisocyanat-Komponente a), eine Polyol-Komponente b), einen Photoinitiator c) und ein Schreibmonomer d), wobei die Photopolymer-Formulierung wenigstens eine Verbindung gemäß der Formel (I) als Schreibmonomer enthält.

Als Polyisocyanat-Komponente a) können alle dem Fachmann bekannten Verbindungen oder deren Mischungen eingesetzt werden, die im Mittel zwei oder mehr NCO-Funktionen pro Molekül aufweisen. Diese können auf aromatischer, araliphatischer, aliphatischer oder cycloaliphatischer Basis sein. In untergeordneten Mengen können auch Monoisocyanate und/oder ungesättigte Gruppen enthaltende Polyisocyanate mitverwendet werden.

Beispielsweise geeignet sind Butylendiisocyanat, Hexamethylendiisocyanat (HDI), Isophorondiisocyanat (IPDI), 1,8-Diisocyanato-4-(isocyanatomethyl)-octan, 2,2,4- und/oder 2,4,4-Trimethylhexamethylendiisocyanat, die isomeren Bis-(4,4'-isocyanatocyclohexyl)methan und deren Mischungen beliebigen Isomerengehalts, Isocyanatomethyl-1,8-octandiisocyanat, 1,4-Cyclohexylendiisocyanat, die isomeren Cyclohexandimethylendiisocyanate, 1,4-Phenylendiisocyanat, 2,4- und/oder 2,6-Toluylendiisocyanat, 1,5-Naphthylendiisocyanat, 2,4'- oder 4,4'-Diphenylmethandiisocyanat und/oder Triphenylmethan-4,4',4"-triisocyanat.

Ebenfalls möglich ist der Einsatz von Derivaten monomerer Di- oder Triisocyanate mit Urethan-, Harnstoff-, Carbodiimid-, Acylharnstoff-, Isocyanurat-, Allophanat-, Biuret-, O-xadiazintrion-, Uretdion- und/oder Iminooxadiazindionstrukturen.

Bevorzugt ist der Einsatz von Polyisocyanaten auf Basis aliphatischer und/oder cycloaliphatischer Di- oder Triisocyanate.

Besonders bevorzugt handelt es sich bei den Polyisocyanaten der Komponente a) um di- oder oligomerisierte aliphatische und/oder cycloaliphatische Di- oder Triisocyanate.

Ganz besonders bevorzugt sind Isocyanurate, Uretdione und/oder Iminooxadiazindione basierend auf HDI, 1,8-Diisocyanato-4-(isocyanatomethyl)-octan oder deren Mischungen.

Ebenfalls können als Komponente a) NCO-funktionelle Prepolymere mit Urethan-, Allophanat-, Biuret- und/oder Amidgruppen eingesetzt werden. Prepolymere der Komponente a) werden in dem Fachmann bekannter Art und Weise durch Umsetzung von monomeren, oligomeren oder Polyisocyanaten a1) mit isocyanatreaktiven Verbindungen a2) in geeigneter Stöchiometrie unter optionalem Einsatz von Katalysatoren und Lösemitteln erhalten.

Als Polyisocyanate a1) eignen sich alle dem Fachmann bekannten aliphatischen, cycloaliphatischen, aromatischen oder araliphatischen Di- und Triisocyanate, wobei es unerheblich ist, ob diese mittels Phosgenierung oder nach phosgenfreien Verfahren erhalten wurden. Daneben können auch die dem Fachmann bekannten höhermolekularen Folgeprodukte monomerer Di- und/oder Triisocyanate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur jeweils einzeln oder in beliebigen Mischungen untereinander eingesetzt werden.

Beispiele für geeignete monomere Di- oder Triisocyanate, die als Komponente a1) eingesetzt werden können, sind Butylendiisocyanat, Hexamethylendiisocyanat (HDI), Isophorondiisocyanat (IPDI), Trimethyl-hexamethylen-diisocyanat (TMDI), 1,8-Diisocyanato-4-(isocyanatomethyl)-octan, Isocyanatomethyl-1,8-octandiisocyanat (TIN), 2,4- und/oder 2,6-Toluen-diisocyanat.

Als isocyanatreaktive Verbindungen a2) für den Aufbau der Prepolymere werden bevorzugt OH-funktionelle Verbindungen eingesetzt. Diese entsprechen den OH-funklionellen Verbindungen, die sie nachfolgend für die Komponente b) beschrieben werden.

Zur Prepolymerherstellung können ebenfalls Amine eingesetzt werden. Beispielsweise geeignet sind hier Ethylendiamin, Diethylentriamin, Triethylentetramin, Propylendiamin, Diaminocyclohexan, Diaminobenzol, Diaminobisphenyl, difunktionelle Polyamine wie z.B. die Jeffamine®, aminterminierte Polymere mit zahlenmittleren Molmassen bis 10000 g/Mol oder deren beliebige Gemische untereinander.

Zur Herstellung von biuretgruppenhaltigen Prepolymeren wird Isocyanat im Überschuss mit Amin umgesetzt, wobei eine Biuretgruppe entsteht. Als Amine eignen sich in diesem Falle für die Umsetzung mit den erwähnten Di-, Tri- und Polyisocyanaten alle oligomeren oder polymeren, primären oder sekundären, difunktionellen Amine der vorstehend genannten Art.

Bevorzugte Prepolymere sind Urethane, Allophanate oder Biurete aus aliphatischen Isocyanatfunktionellen Verbindungen und oligomeren oder polymeren Isocyanat-reaktiven Verbindungen mit zahlenmittleren Molmassen von 200 bis 10000 g/Mol. Besonders bevorzugt sind Urethane, Allophanate oder Biurete aus aliphatischen Isocyanat-funktionellen Verbindungen und oligomeren oder polymeren Polyolen oder Polyaminen mit zahlenmittleren Molmassen von 500 bis 8500 g/Mol. Ganz besonders bevorzugt sind Allophanate aus HDI oder TMDI und difunktionellen Polyetherpolyolen mit zahlenmittleren Molmassen von 1000 bis 8200 g/Mol.

Bevorzugt weisen die vorstehend beschriebenen Prepolymere Restgehalte an freiem monomeren Isocyanat von weniger als 1 Gew.-%, besonders bevorzugt weniger als 0.5 Gew.-% und ganz besonders bevorzugt weniger als 0.2 Gew.-% auf.

Selbstverständlich kann die Isocyanatkomponente anteilsmäßig neben den beschriebenen Prepolymeren weitere Isocyanatkomponenten enthalten. Hierfür kommen aromatische, araliphatische, aliphatische und cycloaliphatische Di-, Tri- oder Polyisocyanate in Betracht. Es können auch Mischungen solcher Di-, Tri- oder Polyisocyanate eingesetzt werden. Beispiele geeigneter Di-, Tri- oder Polyisocyanate sind Butylendiisocyanat, Hexamethylendiisocyanat (HDI), Isophorondiisocyanat (IPDI), 1,8-Diisocyanato-4-(isocyanatomethyl)octan, 2,2,4- und/oder 2,4,4-Trimethylhexamethylendiisocyanat (TMDI), die isomeren Bis(4,4'-isocyanatocyclohexyl)methane und deren Mischungen beliebigen Isomerengehalts, Isocyanatomethyl-1,8-octandiisocyanat, 1,4-Cyclohexylendiisocyanat, die isomeren Cyclohexandimethylendiisocyanate, 1,4-Phenylendiisocyanat, 2,4- und/oder 2,6-Toluylendiisocyanat, 1,5-Naphthylendiisocyanat, 2,4'- oder 4,4'-Diphenylmethandiisocyanat, Triphenylmethan-4,4',4''-triisocyanat oder deren Derivate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind Polyisocyanate auf Basis oligomerisierter und/oder derivatisierter Diisocyanate, die durch geeignete Verfahren von überschüssigem Diisocyanat befreit wurden. Besonders bevorzugt sind die oligomeren Isocyanurate, Uretdione und Iminooxadiazindione des HDI sowie deren Mischungen.

Es ist gegebenenfalls auch möglich, dass die Isocyanatkomponente a) anteilsmäßig Isocyanate enthält, die teilweise mit isocyanat-reaktiven ethylenisch ungesättigten Verbindungen umgesetzt sind. Bevorzugt werden hierbei als isocyanat-reaktive ethylenisch ungesättigte Verbindungen $\alpha,\beta$-ungesättigte Carbonsäurederivate wie Acrylate, Methacrylate, Maleinate, Fumarate, Maleimide, Acrylamide, sowie Vinylether, Propenylether, Allylether und Dicyclopentadienyl-Einheiten enthaltende Verbindungen, die mindestens eine gegenüber Isocyanaten reaktive Gruppe aufweisen, eingesetzt. Besonders bevorzugt sind dies Acrylate und Methacrylate mit mindestens einer isocyanatreaktiven Gruppe. Als hydroxyfunktionelle Acrylate oder Methacrylate kommen beispielsweise Verbindungen wie 2-Hydroxyethyl(meth)acrylat, Polyethylenoxid-mono(meth)acrylate, Polypropylenoxidmono(meth)acrylate, Polyalkylenoxidmono(meth)-acrylate, Poly($\varepsilon$-caprolacton)mono(meth)acrylate, wie z.B. Tone® M100 (Dow, USA), 2-Hydroxypropyl(meth)acrylat, 4-Hydroxybutyl(meth)acrylat, 3-Hydroxy-2,2-dimethylpropyl(meth)acrylat, die hydroxyfunktionellen Mono-, Di- oder Tetra(meth)acrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische in Betracht. Darüberhinaus sind isocyanatreaktive oligomere oder polymere ungesättigte Acrylat- und/oder Methacrylatgruppen enthaltende Verbindungen alleine oder in Kombination mit den vorgenannten monomeren Verbindungen geeignet. Der Anteil an Isocyanaten an der Isocyanatkomponente a), die teilweise mit isocyanat-reaktiven ethylenisch ungesättigten Verbindungen umgesetzt sind beträgt vorzugsweise 0 bis 99 Gew. %, bevorzugt 0 bis 50 Gew.%, besonders bevorzugt 0 bis 25 Gew. % und ganz besonders bevorzugt 0 bis Gew. 15 %.

Es ist auch möglich, dass die vorgenannten Isocyanatkomponente a) vollständig oder anteilsmäßig Isocyanate enthält, die ganz oder teilweise mit dem Fachmann aus der Beschichtungstechnologie bekannten Blockierungsmitteln umgesetzt sind. Als Beispiel für Blockierungsmittel seien genannt: Alkohole, Lactame, Oxime, Malonester, Alkylacetoacetate, Triazole, Phenole, Imidazole, Pyrazole sowie Amine, wie z.B. Butanonoxim, Diisopropylamin, 1,2,4-Triazol, Dimethyl-1,2,4-triazol, Imidazol, Malonsäurediethylester, Acetessigester, Acetonoxim, 3,5-Dimethylpyrazol, $\varepsilon$-Caprolactam, N-tert.-Butyl-benzylamin, Cyclopentanoncarboxyethylester oder beliebige Gemische dieser Blockierungsmittel.

Ganz besonders bevorzugt kann es sich bei der Polyisocyanat-Komponente der erfindungsgemäßen Photopolymer-Formulierung um ein aliphatisches Polyisocyanat oder ein Präpolymer mit primären NCO-Gruppen handeln.

Als Polyolkomponente b) können an sich alle polyfunktionellen, isocyanatreaktiven Verbindungen eingesetzt werden, die im Mittel wenigstens 1,5 isocyanatreaktive Gruppen pro Molekül aufweisen.

Isocyanatreaktive Gruppen im Rahmen der vorliegenden Erfindung sind bevorzugt Hydroxy-, Amino- oder Thiogruppen. Besonders bevorzugt handelt es sich dabei um Hydroxygruppen.

Geeignete polyfunktionelle, isocyanatreaktive Verbindungen sind beispielsweise Polyester-, Polyether-, Polycarbonat-, Poly(meth)acrylat- und/oder Polyurethanpolyole.

Als Polyesterpolyole sind beispielsweise lineare Polyesterdiole oder verzweigte Polyesterpolyole geeignet, wie sie in bekannter Weise aus aliphatischen, cycloaliphatischen oder aromatischen Di- bzw. Polycarbonsäuren bzw. ihren Anhydriden mit mehrwertigen Alkoholen einer OH-Funktionalität $\geq 2$ erhalten werden.

Beispiele für solche Di- bzw. Polycarbonsäuren bzw. Anhydride sind Bernstein-, Glutar-, Adipin-, Pimelin-, Kork-, Azelain-, Sebacin-, Nonandicarbon-, Decandicarbon-, Terephthal-, Isophthal-, o-Phthal-, Tetrahydrophthal-, Hexahydrophthal- oder Trimellitsäure sowie Säureanhydride wie o-Phthal-, Trimellit- oder Bernsteinsäureanhydrid oder deren beliebige Gemische untereinander.

Beispiele für geeignete Alkohole sind Ethandiol, Di-, Tri-, Tetraethylenglykol, 1,2-Propandiol, Di-, Tri-, Tetrapropylenglykol, 1,3-Propandiol, Butandiol-1,4, Butandiol-1,3, Butandiol-2,3, Pentandiol-1,5, Hexandiol-1,6, 2,2-Dimethyl-1,3-propandiol, 1,4-Dihydroxycyclohexan, 1,4-Dimethylolcyclohexan, Octandiol-1,8, Decandiol-1,10, Dodecandiol-1,12, Trimethylolpropan, Glycerin oder deren beliebige Gemische untereinander.

Die Polyesterpolyole können auch auf natürlichen Rohstoffen wie Rizinusöl basieren. Ebenfalls möglich ist, dass die Polyesterpolyole auf Homo- oder Mischpolymerisaten von Lactonen basieren, wie sie bevorzugt durch Anlagerung von Lactonen bzw. Lactongemischen wie Butyrolacton, $\varepsilon$-Caprolacton und/oder Methyl-$\varepsilon$-caprolacton an hydroxyfunktionelle Verbindungen wie mehrwertige Alkohole einer OH-Funktionalität $\geq 2$ beispielsweise der vorstehend genannten Art oder der weiter untern beschriebenen Polyetherpolyole erhalten werden können.

Solche Polyesterpolyole haben bevorzugt zahlenmittlere Molmassen von 400 bis 4000 g/Mol, besonders bevorzugt von 500 bis 2000 g/Mol. Ihre OH-Funktionalität beträgt bevorzugt 1,5 bis 3,5, besonders bevorzugt 1,8 bis 3,0.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die im Rahmen der Polyestersegmente genannten mehrwertigen Alkoholen einer OH-Funktionalität $\geq 2$. Bevorzugt sind 1,4-Butandiol, 1,6-Hexandiol und/oder 3-Methylpentandiol. Polyesterpolyole können auch zu Polycarbonatpolyolen umgearbeitet werden.

Derartige Polycarbonatpolyole haben bevorzugt zahlenmittlere Molmassen von 400 bis 4000 g/Mol, besonders bevorzugt von 500 bis 2000 g/Mol. Die OH-Funktionalität dieser Polyole beträgt bevorzugt 1,8 bis 3,2, besonders bevorzugt 1,9 bis 3,0.

Geeignete Polyetherpolyole sind gegebenenfalls blockweise aufgebaute Polyadditionsprodukte cyclischer Ether an OH- oder NH-funktionelle Startermoleküle.

Geeignete cyclische Ether sind beispielsweise Styroloxide, Ethylenoxid, Propylenoxid, Tetrahydrofuran, Butylenoxid, Epichlorhydrin, sowie ihre beliebigen Mischungen.

Als Starter können die im Rahmen der Polyesterpolyole genannten mehrwertigen Alkohole einer OH-Funktionalität $\geq 2$ sowie primäre oder sekundäre Amine und Aminoalkohole verwendet werden.

Bevorzugte Polyetherpolyole sind solche der vorgenannten Art, die ausschließlich auf Propylenoxid basieren. Vorteilhaft sind auch statistische oder Block-Copolymere basierend auf Propylenoxid mit weiteren 1-Alkylenoxiden, bei denen der 1-Alkylenoxidanteil nicht höher als 80 Gew.-% ist. Daneben sind besonders Poly(tetramethylenoxid)e wie sie z.B. aus der ringöffnenden Polymerisation von Tetrahydrofuran erhalten werden können geeignet sowie Mischungen der als bevorzugt genannten Polyole bevorzugt. Besonders bevorzugt sind Propylenoxidhomopolymere sowie statistische oder Block-Copolymere, die Oxyethylen-, Oxypropylen- und/oder Oxybutyleneinheiten aufweisen, wobei der Anteil der Oxypropyleneinheiten, bezogen auf die Gesamtmenge aller Oxyethylen-, Oxypropylen- und Oxybutyleneinheiten, mindestens 20 Gew.-% und bevorzugt mindestens 45 Gew.-% ausmacht. Oxypropylen- und Oxybutylen umfasst hierbei alle jeweiligen linearen und verzweigten C3- und C4-Isomere.

Derartige Polyetherpolyole haben bevorzugt zahlenmittlere Molmassen von 250 bis 10000 g/Mol, besonders bevorzugt von 500 bis 8500 g/Mol und ganz besonders bevorzugt von 600 bis 4500 g/Mol. Die OH-Funktionalität beträgt bevorzugt 1.5 bis 4.0 und besonders bevorzugt 1.8 bis 3.1.

Daneben sind als Bestandteile der Komponente b) als polyfunktionelle, isocyanatreaktive Verbindungen 2 bis 20 Kohlenstoffatome enthaltende aliphatische, araliphatische oder cycloaliphatische di, tri oder polyfunktionelle Alkohol mit Molekulargewichten kleiner 500 g/mol geeignet.

Dies können beispielsweise Ethylenglykol, Diethylenglykol, Triethylenglykol, Tetraethylenglykol, Dipropylenglykol, Tripropylenglykol, 1,2-Propandiol, 1,3-Propandiol, 1,4-Butandiol, Neopentylglykol, 2-Ethyl-2-butylpropandiol, Trimethylpentandiol, stellungsisomere Diethyloctandiole, 1,3-Butylenglykol, Cyclohexandiol, 1,4-Cyclohexandimethanol, 1,6-Hexandiol, 1,2- und 1,4-Cyclohexandiol, hydriertes Bisphenol A (2,2-Bis(4-hydroxycyclohexyl)propan), 2,2-Dünethyl-3-hydroxypropionsäure (2,2-dimethyl-3-hydroxypropylester) sein. Beispiele geeigneter Triole sind Trimethylolethan, Trimethylolpropan oder Glycerin. Geeignete höherfunktionelle Alkohole sind Ditrimethylolpropan, Pentaerythrit, Dipentaerythrit oder Sorbit.

Besonders bevorzugt ist, wenn bei der erfindungsgemäßen Photopolymer-Formulierung die Polyol-Komponente ein difunktioneller Polyether-, Polyester oder ein Polyether-polyesterblock-copolyester mit primären OH-Funktionen ist.

Die eingesetzten Photoinitiatoren c) sind üblicherweise durch aktinische Strahlung aktivierbare Initiatoren, die eine Polymerisation der entsprechenden polymerisierbaren Gruppen auslösen. Photoinitiatoren sind an sich bekannte, kommerziell vertriebene Verbindungen, wobei zwischen unimolekularen (Typ I) und bimolekularen (Typ II) Initiatoren unterschieden wird. Desweiteren werden diese Initiatoren je nach chemischer Natur für die radikalische, die anionische (oder), die kationische (oder gemischte) Formen der vorgenannten Polymerisationen eingesetzt.

Die Photoinitiatoren c) können insbesondere einen anionischen, kationischen oder neutralen Farbstoff und einen Coinitiator umfassen.

(Typ I)-Systeme für die radikalische Photopolymerisation sind z.B. aromatische Ketonverbindungen, z.B. Benzophenone in Kombination mit tertiären Aminen, Alkylbenzophenone, 4,4'Bis(dimethylamino)benzophenone (Michlers Keton), Anthron und halogenierte Benzophenone oder Mischungen der genannten Typen. Weiter geeignet sind (Typ II)-Initiatoren wie Benzoin und seine Derivate, Benzilketale, Acylphosphinoxide z.B. 2,4,6-Trimethyl-benzoyldiphenylphosphinoxid, Bisacylophosphinoxide, Phenylglyoxylsäureester, Campherchinon, alpha-Aminoalkylphenon, alpha-,alpha-Dialkoxyacetophenon, 1-[4-(Phenylthio)phenyl]octan-1,2-dion-2-(O-benzoyloxim) und alpha-Hydroxyalkylphenon.

Auch die in EP-A 0 223 587 beschriebenen Photoinitiatorsysteme bestehend aus einer Mischung aus einem Ammoniumarylborat und einem oder mehreren Farbstoffen können als Photoinitiator eingesetzt werden. Als Ammoniumarylborat eignen sich beispielsweise Tetrabutylammonium Tetrahexylborat, Tetrabutylammonium Triphenylhexylborat, Tetrabutylammonium Triphenylbutylborat, Tetrabutylammonium Trinapthylhexylborat, Tetrabutylammonium Tris(4-tert.butyl)-phenylbutylborat, Tetrabutylammonium Tris-(3-fluorphenyl)-hexylborat, Tetramethylammonium Triphenylbenzylborat, Tetra(n-hexyl)ammonium (sec-Butyl)triphenylborat, 1-Methyl-3-octylimidazolium Dipentyldiphenylborat und Tetrabutylammonium Tris-(3-Chlor-4-methylphenyl)-hexylborat. Als Farbstoffe eignen sich beispielsweise Neu-Methylenblau, Thionin, Basic Yellow, Pinacynol Chlorid, Rhodamin 6G, Gallocyanin, Ethylviolett, Victoria Blue R, Celestine Blue, Chinaldinrot, Kristallviolett, Brilliant Grün, Astrazon Orange G, Darrow Red, Pyronin Y, Basic Red 29, Pyrillium I, Cyanin und Methylenblau, Azur A (Cunningham et al., RadTech'98 North America UV/EB Conference Proceedings, Chicago, Apr. 19-22, 1998).

Die für die anionische Polymerisation verwendeten Photoinitiatoren sind in der Regel (Typ I)-Systeme und leiten sich von Übergangsmetall-Komplexen der ersten Reihe ab. Hier sind Chrom-Salze, wie z.B. trans-$Cr(NH_3)_2(NCS)_4^-$ (Kutal et al, Macromolecules 1991,24,6872) oder Ferrocenyl-Verbindungen (Yamaguchi et al. Macromolecules 2000, 33, 1152) zu nennen. Eine weitere Möglichkeit der anionischen Polymerisation besteht in der Verwendung von Farbstoffen, wie Kristallviolett Leukonitril oder Malchit Grün Leukonitril, die durch photolytischen Zerfall Cyanoacrylate polymerisieren können (Neckers et al. Macromolecules 2000, 33,7761).

Die für die kationische Polymerisation verwendeten Photoinitiatoren bestehen im wesentlichen aus drei Klassen: Aryldiazonium-Salze, Onium-Salze (hier speziell: Iodonium-, Sulfonium-und Selenonium-Salze) sowie Organometall-Verbindungen. Phenyldiazonium-Salze können unter Bestrahlung sowohl in Gegenwart als auch bei Abwesenheit eines Wasserstoff-Donors ein Kation erzeugten, dass die Polymerisation initiiert. Die Effizienz des Gesamtsystems wird durch die Natur des verwendeten Gegenions zur Diazonium-Verbindung bestimmt. Bevorzugt sind hier die wenig reaktiven aber recht teuren $SbF_6^-$, $AsF_6^-$ oder $PF_6^-$. Für den Einsatz in Beschichtung dünner Filme sind diese Verbindungen i.d.R wenig geeignet, da die den nach der Belichtung freigesetzten Stickstoff die Oberflächegüte herabgesetzt wird (pinholes) (Li et al., Polymeric Materials Science and Engineering, 2001, 84, 139).

Sehr weit verbreitet und auch in vielerlei Form kommerziell erhältlich sind Onium-Salze, speziell Sulfonium- und Iodonium-Salze. Die Photochemie dieser Verbindungen ist nachhaltig untersucht worden. Die Iodonium-Salze zerfallen nach der Anregung zunächst homolytisch und erzeugen somit ein Radikal und ein Radikalanion, welches sich durch H-Abstraktion stabilisiert und ein Proton freisetzt und dann die kationische Polymerisation startet (Dektar et al. J. Org. Chem. 1990, 55, 639; J. Org. Chem., 1991, 56. 1838). Dieser Mechanimus ermöglicht den Einsatz von Iodonium-Salzen ebenfalls für die radikalische Photopolymerisation. Hierbei kommt erneut der Wahl des Gegenions eine große Bedeutung zu, bevorzugt werden ebenfalls $SbF_6^-$, $AsF_6^-$ oder $PF_6^-$. Ansonsten ist in dieser Strukturklasse die Wahl der Substitution des Aromaten recht frei und im wesentlichen durch die Verfügbarkeit geeigneter Startbausteine für die Synthese bestimmt.

Bei den Sulfonium-Salzen handelt es sich um Verbindungen, die in nach Norrish(II) zerfallen (Crivello et al., Macromolecules, 2000, 33, 825). Auch bei den Sulfonium-Salzen kommt der Wahl des Gegenions eine kritische Bedeutung zu, die sich im Wesentlichen in der Härtungsgeschwindigkeit der Polymere äußert. Die besten Ergebnisse werden i.d.R. mit $SbF_6^-$ Salzen erzielt.

Da die Eigenabsorption von Iodonium- und Sulfonium-Salze bei <300nm liegt, müssen diese Verbindungen für die Photopolymerisation mit nahem UV oder kurzwelligem sichtbarem Licht entsprechend sensibilisiert werden. Dies gelingt durch die Verwendung von höher absorbierenden Aromaten wie z.B. Anthracen und Derivaten (Gu et al., Am. Chem. Soc. Polymer Preprints, 2000, 41 (2), 1266) oder Phenothiazin bzw. dessen Derivate (Hua et al, Macromolecules 2001, 34, 2488-2494).

Es kann vorteilhaft sein, Gemische dieser Verbindungen einzusetzen. Je nach zur Härtung verwendeter Strahlungsquelle muss Typ und Konzentration an Photoinitiator in dem Fachmann bekannter Weise angepasst werden. Näheres ist zum Beispiel in P. K. T. Oldring (Ed.), Chemistry & Technology of UV & EB Formulations For Coatings, Inks & Paints, Vol. 3, 1991, SITA Technology, London, S. 61 --- 328 beschrieben.

Bevorzugte Photoinitiatoren c) sind Mischungen aus Tetrabutylammonium Tetrahexylborat, Tetrabutylammonium Triphenylhexylborat, Tetrabutylammonium Triphenylbutylborat, Tetrabutylammonium Trinapthylbutylborat, Tetrabutylammonium Tris-(4-tert.-butyl)-phenylbutylborat, Tetrabutylammonium Tris-(3-fluorphenyl)-hexylborat ([191726-69-9], CGI 7460, Produkt der Ciba Inc, Basel) und Tetrabutylammonium Tris-(3-Chlor-4-methylphenyl)-hexylborat ([1147315-11-4], CGI 909, Produkt der Ciba Inc, Basel) mit Farbstoffen wie beispielsweise Astrazon Orange G, Methylenblau, Neu Methylenblau, Azur A, Pyrillium I, Safranin O, Cyanin, Gallocyanin, Brilliant Grün, Kristallviolett, Ethylviolett und Thionin.

Gemäß einer besonders bevorzugten Ausführungsform der erfindungsgemäßen Photopolymer-Formulierung ist vorgesehen, dass der Photoinitiator aus einer Kombination von Farbstoffen, deren Absorptionsspektren zumindest teilweise den Spektralbereich von 400 bis 800 nm abdecken, mit wenigstens einem geeigneten Coinitiator besteht.

Besonders hohe Brechungsindexkontraste können erzielt werden, wenn die Photopolymer-Formulierung als weiteres Schreibmonomer d) neben den erfindungsmäßen Schreibmonomere der Formel (I) bevorzugt ein acrylat- oder methacrylatfunktionelles Schreibmonomer enthält. Besonders bevorzugt sind dabei monofunktionelle Schreibmonomere, insbesondere diejenigen monofunktionellen Urethan(meth)acrylate wie sie in US 2010/0036013 A1 beschrieben sind.

Eine Steigerung des maximalen Brechungsindexkontrastes kann auch mit einer Photopolymer-Formulierung erzielt werden, die zusätzlich Weichmacher e), bevorzugt Weichmacher gemäß der allgemeinen Formel (II)

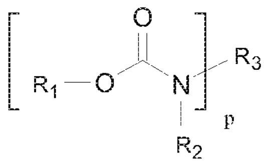

(II)

umfasst, in der p ≥ 1 und ≤ 8 ist und $R^1$, $R^2$, $R^3$ Wasserstoff und / oder unabhängig voneinander lineare, verzweigte, cyclische oder heterocyclische unsubstituierte oder gegebenenfalls auch mit Heteroatomen substituierte organische Reste sind, wobei bevorzugt mindestens einer der Reste $R^1$, $R^2$, $R^3$ mit wenigstens einem Fluoratom substituiert ist und besonders bevorzugt $R^1$ ein organischer Rest mit mindestens einem Fluoratom ist.

Die Weichmacher der Formel II sind Fluorurethane, die durch die Umsetzung eines Biuret, Isocyanurat, Uretdion, Polyharnstoff, Iminooxadiazindione oder Oxadiazadion enthalten Polyisocyanates, das wenigstens eine freie Isocyanat Gruppe aufweist, mit einem Alkohol, wobei das Polyisocyanat und / oder der Alkohol mit wenigstens einem Fluoratom substituiert ist, erhältlich sind.

Die Fluorurethane der Formel (II) sind weiterhin durch Umsetzung von Isocyanaten der Formel $R[NCO]_n$ mit fluorierten Alkoholen in stöchiometrischem Verhältnis zueinander unter Urethanbildung erhältlich.

Bevorzugte Isocyanate der Formel $R[NCO]_n$ sind Methylisocyanat, Ethylisocyanat, die isomeren Propylisocyanate, die isomeren Butylisocyanate, die isomeren Pentylisocyanate, die isomeren Hexylisocyanate, die isomeren Heptylisocyanate, die isomeren Octylisocyanate, die isomeren Nonylisocyanate, die isomeren Decylisocyanate, Stearylisocyanat, Cyclopropylisocyanat, Cyclobulylisocyanat, Cyclopentylisocyanat, Cyclohexylisocyanat, Cycloheptylisocyanat, 2-Methylpentan-1,5-diisocyanat (MPDI), Dodecamethylene-diisocyanat, 1,8-Diisocyanato-4-(isocyanatomethyl)octan (TIN), 6-Diisocyanatohexan (HDI, Desmodur H), 1-Isocyanato-3,3,5-trimethyl-5-isocyanatomethyl-cyclohexan (IPDI, Desmodur I), 2,4,4-Trimethylhexane-1,6-diisocyanat (TMDI), Dicyclohexylmethan-diisocyanat (Desmodur W), Hexahydrotoluylendiisocyanat (H6TDI) 1,3-Bis-(isocyanatomethyl)-cyclohexan, Desmodur LD, Desmodur N 100, Desmodur N3200, Desmodur N3300, Desmodur N3350, Desmodur N3368, Desmodur N3375, Desmodur N3390, Desmodur N3400, Desmodur N3600, Desmodur N3600, Desmodur N3790, Desmodur N3800, Desmodur N3900, Desmodur N50, Desmodur N75, Desmodur NZ1, Desmodur PL340, Desmodur PL350, Desmodur PM76, Desmodur BL3175, Desmodur BL3272, Desmodur BL3370, Desmodur BL3475, Desmodur BL4265, Desmodur BL5375, Desmodur BLXP2677, Desmodur DA-L, Desmodur DN, Desmodur E 305, Desmodur E3265, Desmodur E3370, Baymicron OXA, Desmodur VP LS 2078/2, Desmodur VP LS 2114/1, Desmodur VP LS 2257, Desmodur VP LS 2352/1, Desmodur VP LS 2371, Desmodur VP LS 2376/1, Desmodur XP 2406, Desmodur XP 2489, Desmodur XP 2565, Desmodur XP 2580, Desmodur XP 2599, Desmodur XP 2617, Desmodur XP 2626, Desmodur XP 2675, Desmodur XP 2679, Desmodur XP 2714, Desmodur XP 2730, Desmodur XP 2731, Desmodur XP 2742, Desmodur XP 2748, Desmodur Z 4470 oder deren Mischungen. Besonders bevorzugte Isocyanate der Formel R[NCO]n sind isomeren Propylisocyanate, die isomeren Butylisocyanate, die isomeren Pentylisocyanate, die isomeren Hexylisocyanate, die isomeren Heptylisocyanate, die isomeren Octylisocyanate, die isomeren Nonylisocyanate, die isomeren Decylisocyanate, Stearylisocyanat, 1,8-Diisocyanato-4-(isocyanatomethyl)octan (TIN), 6-Diisocyanatohexan (HDI, Desmodur H), 1-Isocyanato-3,3,5-trimethyl-5-isocyanatomethyl-cyclohexan (IPDI, Desmodur I), 2,4,4-Trimethylhexane-1,6-diisocyanat (TMDI), Dicyclohexylmethan-diisocyanat (Desmodur W), Hexahydrotoluylendiisocyanat (H6TDI), 1,3-Bis-(isocyanatomethyl)-cyclohexan, Desmodur LD, Desmodur N3400, Desmodur N3600, Desmodur N3600, Baymicron OXA oder deren Mischungen.

Ganz besonders bevorzugte Isocyanate der Formel $R[NCO]_n$ sind iso-Propylisocyanat, n-Butylisocyanat, n-Hexylisocyanat, n-Octylisocyanat, n-Decylisocyanat, Cyclohexylisocyanat, Stearylisocyanat, 1,8-Diisocyanato-4-(isocyanatomethyl)octan (TIN), 6-Diisocyanatohexan (HDI, Desmodur H), 1-Isocyanato-3,3,5-trimethyl-5-isocyanatomethyl-cyclohexan (IPDI, Desmodur I), 2,4,4-Trimethylhexane-1,6-diisocyanate (TMDI), Dicyclohexylmethan-diisocyanat (Desmodur W), Hexahydrotoluylendiisocyanat (H6TDI), 1,3-Bis-(iso-cyanatomethyl)-cyclohexan, Desmodur LD, Desmodur N3400, Desmodur N3600, Desmodur N3900, Baymicron OXA oder deren Mischungen.

Die Auswahl der fluorierten Alkohole ist dabei breit möglich, es ist bevorzugt, primäre oder sekundäre, mono-, di-, oder trifunktionelle Alkohole mit einem Fluorgehalt von 30 % bis 82 % Fluor, besonders bevorzugt von mit einem Fluorgehalt von 40 % bis 80 % Fluor und insbesondere bevorzugt mit einem Fluorgehalt von 49 % bis 75 % Fluor zu verwenden.

Bei der Umsetzung von Isocyanaten mit Alkoholen der jeweils vorstehend genannten Art zur Herstellung der Fluorurethane (II) handelt es sich um eine Urethanisierung. Die Umsetzung kann unter zu Hilfenahme der zur Beschleunigung von Isocyanatadditionsreaktionen bekannten Katalysatoren, wie z.B. tertiärer Amine, Zinn-, Zink-, Eisen- oder Bismuthverbindungen, insbesondere Triethylamin, 1,4-Diazabicycl-[2,2,2]-octan, Bismuthoctoat, Zinkoctoat oder Dibutylzinndilaurat erfolgen, die mit vorgelegt oder später zudosiert werden können.

Die Fluorurethane der Formel (II) haben einen Gehalt an Isocyanatgruppen (M = 42 g/mol) oder freien Isocyanatrestmonomeren von unter 0.5 Gew.%, bevorzugt unter 0.2 Gew.-%, besonders bevorzugt unter 0.1 Gew.%.

Weiterhin enthalten die Fluorurethane der Formel (II) Gehalte an nicht umgesetzten hydroxyfunktionellen Verbindungen von unter 1 Gew.-%, bevorzugt unter 0.5 Gew.-% und besonders bevorzugt unter 0.2 Gew.-% und weisen einen Fluorgehalt von 10-80 Gew.-% Fluor, bevorzugt von 12.5-75 Gew.-% Fluor, besonders bevorzugt von 15-70 Gew.-% Fluor und insbesondere bevorzugt 17.5-65 Gew.-% Fluor auf. Dabei weisen diese einen Brechungsindex $n_D^{20}$ von $\leq$ 1.4600, bevorzugt von $\leq$ 1.4500, besonders bevorzugt von $\leq$ 1.4400 und insbesondere bevorzugt von $\leq$ 1.4300 auf.

Gemäß einer weiteren bevorzugten Ausführungsform der Erfindung ist vorgesehen, dass die Photopolymer-Formulierung 10 bis 89,999 Gew.-%, bevorzugt 20 bis 70 Gew.-% Matrixpolymere bestehend aus Verbindungen der Komponente a) und der Komponente b), 10 bis 60 Gew.-%, bevorzugt 25 bis 50 Gew.-% Schreibmonomere, 0,001 bis 5 Gew.-% Photoinitiatoren und gegebenenfalls 0 bis 4 Gew.-%, bevorzugt 0 bis 2 Gew.-% Katalysatoren, 0 bis 5 Gew.-% , bevorzugt 0,001 bis 1 Gew.-% Radikalstabilisatoren, 0 bis 40 Gew.-%, bevorzugt 10 bis 30 Gew.-% Weichmacher und 0 bis 5 Gew.-%, bevorzugt 0,1 bis 5 Gew.-% weitere Additive enthält, wobei die Summe aller Bestandteile 100 Gew.-% beträgt.

Besonders bevorzugt werden Photopolymer-Formulierungen mit 20 bis 70 Gew.-% Matrixpolymeren bestehend aus Verbindungen der Komponente a) und der Komponente b), 25 bis 50 Gew.-% Schreibmonomere, 0,001 bis 5 Gew.-% Photoinitiatoren, 0 bis 2 Gew.-% Katalysatoren, 0,001 bis 1 Gew.-% Radikalstabilisatoren gegebenenfalls 10 bis 30 Gew.-% der oben beschriebenen Fluorurethaue der Formel II und gegebenenfalls 0,1 bis 5 Gew.-% weiterer Additive eingesetzt.

Ein weiterer Gegenstand der Erfindung ist die Verwendung einer erfindungsgemäßen Photopolymer-Formulierung zur Herstellung holographischer Medien insbesondere zur Aufzeichnung von In-Line, Off-Axis, Full-Aperture Transfer, Weißlicht-Transmissions, Denisyuk, Off-Axis Reflektions oder Edge-Lit Hologrammen sowie holographischen Stereogrammen.

Noch ein Gegenstand der Erfindung ist die Verwendung einer erfindungsgemäßen Photopolymer-Formulierung zur Herstellung holographischer Medien, die durch entsprechende Belichtungsprozesse für optische Anwendungen im gesamten sichtbaren und nahen UV-Bereich (300-800 nm) zu Hologrammen verarbeitet werden können. Visuelle Hologramme umfassen alle Hologramme, die nach dem Fachmann bekannten Verfahren aufgezeichnet werden können. Darunter fallen unter anderem In-Line (Gabor) Hologramme, Off-Axis Hologramme, Full-Aperture Transfer Hologramme, Weißlicht-Transmissionshologramme ("Regenbogenhologramme), Denisyukhologramme, Off-Axis Reflektionshologramme, Edge-Lit Hologramme sowie holographische Stereogramme. Bevorzugt sind Reflexionshologramme, Edge-Lit Hologramme, Transmissionshologramme.

Mögliche optische Funktionen der Hologramme, die mit den erfindungsgemäßen Photopolymer-Formulierungen hergestellt werden können entsprechen den optische Funktionen von Lichtelementen wie Linsen, Spiegel, Umlenkspiegel, Filter, Streuscheiben, Beugungselemente, Lichtleiter, Lichtlenker (waveguides), Projektionsscheiben und/oder Masken. Zudem können mehrere derartiger optischer Funktionen in einem solchen Hologramm kombiniert werden, z.B. so dass je nach Lichteinfall das Licht in eine andere Richtung abgebeugt wird. So kann man mit derartigen Aufbauten autostereoskopische elektronische Displays bauen, die es erlauben einen stereoskopischen visuellen Eindruck ohne

weitere Hilfsmittel wie z.B. einer Polarisator- oder Shutterbrille zu erleben.

Häufig zeigen diese optischen Elemente eine spezifische Frequenzselektivität, je nachdem wie die Hologramme belichtet wurden und welche Dimensionen das Hologramm hat. Dies ist insbesondere wichtig, wenn man monochromatische Lichtquellen wie LED oder Laserlicht verwendet. So benötigt man ein Hologramm pro Komplementärfarbe (RGB), um Licht frequenzselektiv zu lenken und gleichzeitig vollfarbige Displays zu ermöglichen. Daher sind in bestimmten Displayaufbauten mehrer Hologramme ineinander in einen Photopolymerfilm zu belichten.

Zudem können mittels der erfindungsgemäßen Photopolymer-Formulierungen auch holographische Bilder oder Darstellungen, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können u.a. auch in Kombination mit den zuvor dargestellten Produkten hergestellt werden. Holographische Bilder können den Eindruck eines dreidimensionalen Bildes haben, sie können aber auch Bildsequenzen, kurze Filme oder eine Anzahl von verschiedenen Objekten darstellen, je nachdem aus welchem Winkel, mit welcher (auch bewegten) Lichtquelle etc. diese beleuchtet wird. Aufgrund dieser vielfältigen Designmöglichkeiten stellen Hologramme, insbesondere Volumenhologramme eine attraktive technische Lösung für die oben genannten Anwendung dar.

Die Photopolymer-Formulierungen können insbesondere als holografisches Medium in Form eines Films verwendet werden. Dabei wird als Träger eine Lage eines für Licht im sichtbaren Spektralbereich (Transmission größer als 85% im Wellenlängenbereich von 400 bis 780 nm) transparenten Materials oder Materialverbunds ein- oder beidseitig beschichtet sowie ggf. eine Abdeckschicht auf der oder den Photopolymerlagen appliziert.

Bevorzugte Materialien oder Materialverbünde des Trägers basieren auf Polycarbonat (PC), Polyethylenterephthalat (PET), Polybutylenterephthalat, Polyethylen, Polypropylen, Celluloseacetat, Cellulosehydrat, Cellulosenitrat, Cycloolefinpolymere, Polystyrol, Polyepoxide, Polysulfon, Cellulosetriacetat (CTA), Polyamid, Polymethylmethacrylat, Polyvinylchlorid, Polyvinylbutyral oder Polydicyclopentadien oder deren Mischungen. Besonders bevorzugt basieren sie auf PC, PET und CTA. Materialverbünde können Folienlaminate oder Coextrudate sein. Bevorzugte Materialverbünde sind Duplex- und Triplexfolien aufgebaut nach einem der Schemata A/B, A/B/A oder A/B/C. Besonders bevorzugt sind PC/PET, PET/PC/PET und PC/TPU (TPU = Thermoplastisches Polyurethan).

Alternativ zu den vorgenannten Kunststoffträgern können auch planare Glasplatten eingesetzt werden, die insbesondere für großflächige abbildungsgenaue Belichtungen, z.B. für holographische Lithographie (Ng, Willie W.; Hong, Chi-Shain; Yariv, Amnon. Holographic interference lithography for integrated optics. IEEE Transactions on Electron Devices (1978), ED-25(10), 1193-1200, ISSN:0018-9383) Verwendung finden.

Die Materialien oder Materialverbünde des Trägers können einseitig oder beidseitig antihaf tend, antistatisch, hydrophobiert oder hydrophiliert ausgerüstet sein. Die genannten Modifikationen dienen an der Photopolymerschicht zugewandten Seite dem Zweck, dass die Photopolymerlage von dem Träger zerstörungsfrei abgelöst werden kann. Eine Modifikation der der Photopolymerlage abgewandten Seite des Trägers dient dazu, dass die erfindungsgemäßen Medien speziellen mechanischen Anforderungen genügen, die z.B. bei der Verarbeitung in Rollenlaminatoren, insbesondere bei Rolle-zu-Rolle-Verfahren, gefordert sind.

## Beispiele

Die Erfindung wir im Folgenden anhand von Beispielen näher erläutert.

In der Zeichnung zeigt

Figur 1 die Geometrie eines Holographic Media Testers (HMT) bei $\lambda$ = 532 nm (DPSS Laser = Diode Pumped Solid State Laser) und

Figur 2 die gemessene Beugungseffizienz $\eta$ als Kreise gegen das Winkeldetuning $\Delta\Omega$ aufgetragen und die Anpassung der Kogelnik Theorie als durchgezogene Linie. Die Darstellung zeigt Vergleichsbeispiel 2

## Messmethoden:

### Brechungsindezbestimmung:

Die Messung des Brechungsindex erfolgte für hochviskose und feste Produkte bei einer Wellenlänge von 589 nm indem der Brechungsindex n in Abhängigkeit von der Wellenlänge der Probe aus den Transmissions- und Reflexionsspektren erhalten wird. Dazu wurden ca. 100 - 300 nm dicke Filme der Proben auf Quarzglasträger aus einer fünf gewichtsprozentigen Lösung in Ethylacetat aufgeschleudert. Das Transmissions- und Reflexionsspektrum dieses Schichtpaketes wurde mit einem Spektrometer der Firma STEAG ETA-Optik, CD-Measurement System ETA-RT gemessen und danach die Schichtdicke und der spektrale Verlauf von n an die gemessenen Transmissions- und Reflexionsspektren angepasst. Dies geschieht mit der internen Software des Spektrometers und erfordert zusätzlich die n Daten des Quarzglassubstrates, die in einer Blindmessung vorab bestimmt wurden.

Für flüssige Produkte wird ein Abbe-Refraktrometer zur Brechungsindexbestimmung bei 589 nm verwendet. Dabei wurden 3 Tropfen des Produktes auf das gesäuberte Meßprisma des Geräts aufgebracht, das Beleuchtungsprisma zugeklappt und innerhalb von 2 Minuten auf 20°C temperiert. Anschließend wurde im Beobachtungsfeld die Hell-Dunkel-Grenze scharf auf das Fadenkreuz des Refraktometers eingestellt. Wenn sich der eingestellte Wert nicht mehr änderte, wurde an der Skala im Gerat die Brechzahl auf vier Stellen nach dem Komma abgelesen. Es wurde eine Doppelbestimmung durchgeführt. Abweichungen bis zu 0,0002 Skalenteile waren zulässig.

**Trübungsmessung**

Die Trübungsmessung erfolgte gemäß ASTM D 1003. Die Trübung stellt den prozentualen Anteil durchgelassenen Lichtes dar, der vom eingestrahlten Lichtbündel im Mittel um mehr als 2.5° abweicht. Zur Messung der Trübung wurden die holografischen Coupons vor der Messung außen gereinigt, um eine Verfälschung des Ergebnisses durch Fingerabdrücke und Schmutz auf den Gläsern zu vermeiden. Dann wurden die Coupons in ein Haze-Gard-Plus Gerät der Byk-Gardner zur Vermessung eingesetzt.

**Isocyanatgehalt**

Die angegebenen NCO-Werte (Isocyanat-Gehalte) wurden gemäß DIN EN ISO 11909 bestimmt.

Der vollständige Umsatz von NCO-Gruppen bzw. deren Abwesenheit in einem Reaktionsgemisch wurde IR-spektroskopisch nachgewiesen. So wurde wurde dann ein vollständiger Umsatz angenommen, wenn im IR-Spektrum des Reaktionsgemisches keine NCO-Bande (2261cm$^{-1}$) auftauchte.

**Festkörpergehalt**

Von einem unlackierten Dosendeckel und einer Büroklammer wurde das Tara-Gewicht ermittelt. Dann wurden ca. 1g der zu untersuchenden Probe nach der Einwaage in dem Dosendeckel mit der geeignet gebogenen Büroklammer gleichmäßig verteilt. Die Büroklammer verblieb zur Messung in der Probe. Die Einwaage wurde ermittelt, danach für 1 Stunde bei 125 °C in einen Laborofen erhitzt und anschließend die Auswaage bestimmt. Den Festkörpergehalt wurde gemäß folgender Gleichung bestimmt: Auswaage [g] *100 / Einwaage [g] = Gew.% Festkörper.

**Messung der holographischen Eigenschaften DE und $\Delta$n der holographischen Medien mittels Zweistrahlinterferenz in Transmissionsanordnung**

Die hergestellten Medien wurden mittels einer Messanordnung gemäß Figur 1 wie folgt auf ihre holographischen Eigenschaften geprüft:

> Figur 1 zeigt den holographischen Versuchsaufbau, mit dem die Beugungseffizienz (DE) der Medien gemessen wurde, wobei folgendes gilt: M = Spiegel, S = Verschluss, SF = Raumfilter, CL = Kollimatorlinse, $\lambda/2$ = $\lambda/2$ Platte, PBS = polarisationsempfindlicher Strahlteiler, D = Detektor, I = Irisblende, $\alpha_0$ = -22.3°, $\beta_0$ = 22.3° sind die Einfallswinkel der kohärenten Strahlen außerhalb der Probe (des Mediums) gemessen. RD = Referenzrichtung des Drehtisches.

Der Strahl eines DPSS Lasers (Emissionswellenlänge 532 nm) wurde mit Hilfe des Raumfilter (SF) und zusammen mit der Kollimationslinse (CL) in einen parallelen homogenen Strahl umgewandelt. Die finalen Querschnitte des Signal und Referenzstrahls werden durch die Irisblenden (I) festgelegt. Der Durchmesser der Irisblendenöffnung beträgt 0.4 cm. Die polarisationsabhängigen Strahlteiler (PBS) teilen den Laserstrahl in zwei kohärente gleich polarisierte Strahlen. Über die $\lambda/2$ Plättchen wurden die Leistung des Referenzstrahls auf 2.0 mW und die Leistung des Signalstrahls auf 2.0 mW eingestellt. Die Leistungen wurden mit den Halbleiterdetektoren (D) bei ausgebauter Probe bestimmt. Der Einfallswinkel ($\alpha_0$) des Referenzstrahls beträgt -22.3°, der Einfallswinkel ($\beta_0$) des Signalstrahls beträgt 22.3°. Die Winkel werden ausgehend von der Probennormale zur Strahlrichtung gemessen. Gemäß Figur 1 hat daher $\alpha_0$ ein negatives Vorzeichen und $\beta_0$ ein positives Vorzeichen. Am Ort der Probe (Medium) erzeugte das Interferenzfeld der zwei überlappenden Strahlen ein Gitter heller und dunkler Streifen die parallel zur Winkelhalbierenden der zwei auf die Probe einfallenden Strahlen liegen (Transmissionshologramm). Der Streifenabstand A, auch Gitterperiode genannt, im Medium beträgt ~ 700 nm (der Brechungsindex des Mediums zu ~1.504 angenommen).

Es wurden auf folgende Weise Hologramme in das Medium geschrieben:

- Beide Shutter (S) sind für die Belichtungszeit *t* geöffnet.
- Danach wurde bei geschlossenen Shuttern (S) dem Medium 5 Minuten Zeit für die Diffusion der noch nicht polymerisierten Schreibmonomere gelassen.

Die geschriebenen Hologramme wurden nun auf folgende Weise ausgelesen. Der Shutter des Signalstrahls blieb geschlossen. Der Shutter des Referenzstrahls war geöffnet. Die Irisblende des Referenzstrahls wurde auf einen Durchmesser < 1 mm geschlossen. Damit erreichte man, dass für alle Drehwinkel ($\Omega$) des Mediums der Strahl immer vollständig im zuvor geschriebenen Hologramm lag. Der Drehtisch überstrich nun computergesteuert den Winkelbereich von $\Omega_{min}$ bis $\Omega_{max}$ mit einer Winkelschrittweite von 0.05°. $\Omega$ wird von der Probennormale zur Referenzrichtung des Drehtisches gemessen. Die Referenzrichtung ($\Omega$ = 0) des Drehtisches ergibt sich dann, wenn beim Schreiben des Hologramms der Einfallswinkel des Referenz- und des Signalstrahls betragsmäßig gleich sind also $\alpha_0$ = -22.3° und $\beta_0$ = 22.3° gilt. Allgemein gilt für das Interferenzfeld beim Schreiben ("recording") eines symmetrischen Transmissionshologramms ($\alpha_0$ = - $\beta_0$):

$$\alpha_0 = \theta_0$$

$\theta_0$ ist der Halbwinkel im Laborsystem außerhalb des Mediums. In diesem Fall gilt also $\theta_0$ = - 22.3°. An jedem angefahrenen Drehwinkel $\Omega$ wurden die Leistungen des in der nullten Ordnung transmittierten Strahls mittels des entsprechenden Detektors D und die Leistungen des in die erste Ordnung abgebeugten Strahls mittels des Detektors D gemessen. Die Beugungseffizienz ergab sich bei jedem angefahrenen Winkel $\Omega$ als der Quotient aus:

$$\eta = \frac{P_D}{P_D + P_T}$$

$P_D$ ist die Leistung im Detektor des abgebeugten Strahls und $P_T$ ist die Leistung im Detektor des transmittierten Strahls.

Mittels des oben beschriebenen Verfahrens wurde die Braggkurve, sie beschreibt den Beugungswirkungsgrad $\eta$ in Abhängigkeit des Drehwinkels $\Omega$□ des geschriebenen Hologramms gemessen und in einem Computer gespeichert. Zusätzlich wurde auch die in die nullte Ordnung transmittierte Intensität gegen den Drehwinkel $\Omega$ aufgezeichnet und in einem Computer gespeichert.

Die zentrale Beugungseffizienz (DE = $\eta$0) des Hologramms, wurde bei Q = 0 ermittelt.

Der Brechungsindexkontrast $\Delta$n und die Dicke d der Photopolymerschicht wurde nun mittels der Coupled Wave Theorie (siehe; H. Kogelnik, The Bell System Technical Journal, Volume 48, November 1969, Number 9 Seite 2909 - Seite 2947) an die gemessene Braggkurve angepasst. Das Auswerteverfahren wird im Folgenden beschrieben:

Für die Braggkurve $\eta(\Omega)$ eines Transmissionshologramms gilt nach Kogelnik:

$$\eta = \frac{\sin^2\left(\sqrt{\nu^2 + \xi^2}\right)}{1 + \frac{\xi^2}{\nu^2}}$$

mit:

$$\nu = \frac{\pi \cdot \Delta n \cdot d}{\lambda \cdot \sqrt{|c_s \cdot c_r|}}$$

$$\xi = -\frac{d}{2 \cdot c_s} \cdot DP$$

$$c_s = \cos(\vartheta)$$

$$c_r = \cos(\vartheta)$$

$$DP = \frac{\pi}{\Lambda} \cdot \left( -2 \cdot \sin(\vartheta) - \frac{\lambda}{n \cdot \Lambda} \right)$$

$$\Lambda = -\frac{\lambda}{2 \cdot n \cdot \sin(\alpha)}$$

Beim Auslesen des Hologramms ("reconstruction") gilt wie analog oben dargestellt:

$$\vartheta_0 = \theta_0 + \Omega$$

$$\sin(\vartheta_0) = n \cdot \sin(\vartheta)$$

An der Bragg-Bedingung ist das "Dephasing" DP = 0. Und es folgt entsprechend:

$$\alpha_0 = \theta_0$$

$$\sin(\alpha_0) = n \cdot \sin(\alpha)$$

v ist die Gitterstärke und $\xi$ ist der Detuning Parameter des Brechungsindexgitters das geschrieben wurde. n ist der mittlere Brechungsindex des Photopolymers und wurde zu 1.504 gesetzt. $\lambda$ ist die Wellenlänge des Laserlichts im Vakuum

Die zentrale Beugungseffizienz (DE = $\eta 0$) ergibt sich dann für $\xi = 0$ zu:

$$DE = \sin^2(\nu) = \sin^2\left( \frac{\pi \cdot \Delta n \cdot d}{\lambda \cdot \cos(\alpha)} \right)$$

Die Messdaten der Beugungseffizienz und die theoretische Braggkurve werden wie in Figur 2 gezeigt gegen den Drehwinkel $\Omega$ aufgetragen.

Da DE bekannt ist wird die Form der theoretischen Braggkurve nach Kogelnik nur noch durch die Dicke *d* der Photopolymerschicht bestimmt. $\Delta n$ wird über DE für gegebene Dicke *d* so nachkorrigiert, dass Messung und Theorie von DE immer übereinstimmen, d wird nun solange angepasst bis die Winkelpositionen der ersten Nebenminima und die Höhen der ersten Nebenmaxima der theoretischen Braggkurve mit den Winkelpositionen der ersten Nebenminima und den Höhen der ersten Nebenmaxima der gemessenen Braggkurve übereinstimmen.

Figur 2 zeigt die theoretisch berechnete und an die experimentellen Daten gefittete Braggkurve $\eta$ nach der Coupled Wave Theorie (auch Kogelnik-Theorie genannt) als durchgezogene Linie und zeigt vergleichend den experimentell bestimmten Beugungswirkungsgrades (in Kreissymbolen) aufgetragen gegen den Drehwinkel $\Omega$.

Für eine Formulierung wurde diese Prozedur eventuell mehrfach für verschiedene Belichtungszeiten t an verschiedenen Medien wiederholt, um festzustellen bei welcher mittleren Energiedosis des einfallenden Laserstrahls beim Schreiben des Hologramms $\Delta n$ in den Sättigungswert übergeht. Die mittlere Energiedosis E ergibt sich wie folgt aus den Leistungen der zwei den Winkeln $\alpha_0$ und $\beta_0$ zugeordneten Teilstrahlen (Referenzstrahl mit $P_r$ = 2.00 mW und Signalstrahl mit $P_s$ = 2.00 mW), der Belichtungszeit t und dem Durchmesser der Irisblende (0.4 cm):

$$E\,(\text{mJ/cm}^2) = \frac{2 \cdot [P_r + P_s] \cdot t\,(\text{s})}{\pi \cdot 0.4^2\ \text{cm}^2}$$

**Chemikalien:**

In eckigen Klammern ist jeweils, soweit bekannt die CAS-Nummer angegeben.

**Erfindungsgemäße Schreibmonomere:**

| | |
|---|---|
| 2-[(Biphenyl-2-yloxy)methyl]oxiran | [7144-65-2] - American Custom Chemicals Corporation, San Diego Ca, USA |
| 2-Isocyanatoethylacrylat | [13641-96-8] - Karenz® AOI, SHOWA DENKO K.K., Fine Chemicals Group, Specialty Chemicals Department, Chemicals Division, Japan |
| 1,2-Ethandithiol | [540-63-6] - Merck Schuchardt OHG, Hohenbrunn, Deutschland |
| 1,3-Propandithiol | [109-80-8] - Merck Schuchardt OHG, Hohenbrunn, Deutschland |
| 1,4-Butandithiol | [1191-08-8] - SAFC, St. Louis, USA |
| 2,3-Butandithiol | [4532-64-3] - Sigma-Aldrich Chemie GmbH, Steinheim, Deutschland |
| 1,6-Hexandithiol | [1191-43-1] - SAFC, St. Louis, USA |
| 2,2'-Oxydiethanthiol | [2150-02-9] - ABCR GmbH & Co KG, Karlsruhe, Deutschland |
| 2,2'-Sulfanediyldiethanthiol | DMDES - Toyo Kasei Kogyo Co. Ltd, Takasago-City, Hyogo, Japan |
| 1,2-Bis(2-Mercaptoethoxy)ethan | [14970-87-7] - Sigma-Aldrich Chemie GmbH, Steinheim, Deutschland |
| 1,10-Decandithiol | [1191-67-9] - ABCR GmbH & Co KG, Karlsruhe, Deutschland |
| Ethylenglycoldimercaptoacetat | [123-81-9] - Merck Schuchardt OHG, Hohenbrunn, Deutschland |
| 4,4'-Sulfanediyldibenzolthiol | [19362-77-7] - Sigma-Aldrich Chemie GmbH, Steinheim, Deutschland |
| 2,3-Disulfanylpropan-1-ol | [59-52-9] - Acros Organics, New Jersey, USA |
| 1-Isocyanato-2-(phenylsulfanyl)benzol | [13739-55-4] - APAC Pharmceuticals, Columbia MD, USA |
| 1-Isocyanato-3-(methylsulfanyl)benzol | [28479-19-8]- Sigma-Aldrich Chemie GmbH, Steinheim, Deutschland |
| Tris(p-isocyanatophenyl)thiophosphat | Desmodur® RFE, Produkt der Bayer MaterialScience AG, Leverkusen, Deutschland |
| 3-Phenylphenol | [580-51-8] - Acros Organics, New Jersey, USA |
| Kaliumcarbonat | Sigma-Aldrich Chemie GmbH, Steinheim, Deutschland |
| Epibromhydrin | [3132-64-7]- Sigma-Aldrich Chemie GmbH, Steinheim, Deutschland |
| Triphenylphosphin | [603-35-0] ABCR GmbH & Co KG, Karlsruhe, Deutschland |
| 1-Butyl-3-Methylimidazoliumbromid | [85100-77-2] - ABCR GmbH & Co KG, Karlsruhe, Deutschland |
| Dibutylzinndilaurat | [77-58-7] - Urethanisierungskatalysator Desmorapid Z, Bayer MaterialScience AG, Leverkusen, Deutschland |
| Fomrez® UL 28 | Urethanisierungskatalysator, Handelsprodukt der Momentive Performance Chemicals, Wilton, CT, USA. |
| Addocat® SO | ein Zinn basierender Katalysator der Rhein-Chemie, Mannheim, Deutschland |
| Desmodur® N 3900 | Produkt der Bayer MaterialScience AG, Leverkusen, DE, Hexandiisocyanatbasiertes Polyisocyanat, Anteil an Iminooxadiazindion mindestens 30 %, NCO-Gehah: 23.5 %. |
| CGI-909 | Tetrabutylammonium-tris(3-chlor-4-methylphenyl)(hexyl)borat, [1147315-11-4] ist ein Produkt der BASF SE (ehem. Ciba Inc.). |
| Trimethylhexamethylendiisocyanat | [28679-16-5] - ABCR GmbH & Co KG, Karlsruhe, Deutschland |
| 1H,1H-7H-Perfluorheptan-1-ol | [335-99-9] - ABCR GmbH & Co KG, Karlsruhe, Deutschland |
| Kristallviolett | [548-62-9] Sigma-Aldrich Chemie GmbH, Steinheim, Deutschland |
| Irgacure® 250 | [344562-80-7], Iodonium, (4-methylphenyl)[4-(2-methylpropyl) phenyl]-, hexafluorophosphate(1-) Produkt der BASF SE (ehem Ciba Inc.). |

**Allgemeine Vorschrift zur Herstellung der Dithiol - Oxiran Addukte (Beispiel 1a-12a)**

Das Oxiran und der Katalysator wurden in einem Dreihalskolben vorgelegt, der mit KPG-Rührer und Rührmotor

sowie Trockenrohr ausgerüstet ist. Es wurde auf 60 bis 80°C temperiert und das Dithiol zugetropft. Dann wurde bei der angegebenen Temperatur weitergerührt, bis im $^1$H-NMR-Spektrum ein Umsatz der Oxirangruppe >95% erkennbar war bzw. keine Oxirangruppen mehr nachweisbar waren.

| | |
|---|---|
| **Beispiel 1a** | 3,3'-(Ethan-1,2-diyldisulfanediyl)bis[1-(biphenyl-2-yloxy)propan-2-ol] |
| Edukte: | 14,3g 2-[(Biphenyl-2-yloxy)methyl]oxiran |
| | 34mg 1-Butyl-3-Methylimidazoliumbromid |
| | 2,8g 2-Ethandithiol |
| Bedingungen: | Reaktionstemperatur 70 °C; Addition des Dithiols innerhalb 1 Stunde, Reaktionszeit 19 Stunden |
| | Es wurde eine klare, farblose, viskose Flüssigkeit erhalten. |
| $^1$H-NMR | ($CDCl_3$, 400 MHz): δ (1H)= 7,45 (d, 2H), 7,38 (t, 2H), 7,32 (m, 3H), 7,16 (t, 1H), 6,97 (d, 1H), 3,90-4,05 (m, 3H), 2,60-2,75 (m, 3H), 2,55 (dd, 1H), 2,45 (t, 1H, OH). |

| | |
|---|---|
| **Beispiel 2a** | 3,3'-(Propan-1,3-diyldisulfanediyl)bis[1-(biphenyl-2-yloxy)propan-2-ol] |
| Edukte: | 14,3 g 2-[(Biphenyl-2-yloxy)methyl]oxiran |
| | 33 mg 1-Butyl-3-Methylimidazoliumbromid |
| | 3,2 g 1,3-Propandithiol |
| Bedingungen: | Zugabe innerhalb von 30 Minuten bei 60 °C dann 80 °C, Reaktionszeit 20 h |
| | Es wurde eine klare, farblose, viskose Flüssigkeit erhalten. |
| $^1$H-NMR | ($CDCl_3$, 400 MHz): δ (1H)= 7,45 (d, 4H), 7,38 (t, 6H), 7,32 (m, 6H), 7,16 (t, 2H), 6,97 (d, 2H), 3,98 (d, 4H), 3,92 (m, 2H), 2,64 (dd, 2H), 2,48-2,59 (m, 8H), 1,57 (p, 2H) |

| | |
|---|---|
| **Beispiel 3a** | 3,3'-(Butan-1,4-diyldisulfanediyl)bis[1-(biphenyl-2-yloxy)propan-2-ol] |
| Edukte: | 16,7 g 2-[(Biphenyl-2-yloxy)methyl]oxiran |
| | 42 mg 1-Butyl-3-Methylimidazoliumbromid |
| | 4,3 g 1,4-Butandithiol |
| Bedingungen: | Reaktionstemperatur 80 °C, Reaktionszeit 48,5 h |
| | Es wurde eine klare, farblose, viskose Flüssigkeit erhalten. |
| $^1$H-NMR | ($CDCl_3$, 400 MHz): δ (1H)= 7,45 (d, 2H), 7,38 (t, 2H), 7,32 (m, 3H), 7,16 (t, 1H), 6,97 (d, 1H), 4,05 (d, 2H), 3,95 (m, 1H), 2,65 (dd, 1H), 2,58 (dd, 1H), 2,4-2,55 (m, 2H), 1,5-1,65 (m, 2H) |

| | |
|---|---|
| **Beispiel 4a** | 3,3'-(Butan-2,3-diyldisulfanediyl)bis[1-(biphenyl-2-yloxy)propan-2-ol] |
| Edukte: | 14,3 g 2-[(Biphenyl-2-yloxy)methyl]oxiran |
| | 36 mg 1-Butyl-3-Methylimidazoliumbromid |
| | 3,7 g 2,3-Butandithiol |
| Bedingungen: | Reaktionstemperatur 60 °C beim Zutropfen, 80 °C, Reaktionszeit 48,5 h |
| | Es wurde eine klare, farblose, viskose Flüssigkeit erhalten. |
| $^1$H-NMR | ($CDCl_3$, 400 MHz): δ (1H)= 7,45 (d, 2H), 7,38 (t, 2H), 7,32 (m, 3H), 7,16 (t, 1H), 6,97 (d, 1H), 4,05 (d, 2H), 3,95 (m, 1H), 2,65 (dd, 1H), 2,58 (dd, 1H), 2,4-2,55 (m, 2H), 1,5-1,65 (m, 2H) |

| | |
|---|---|
| **Beispiel 5a** | 3,3'-(Hexan-1,6-diyldisulfanediyl)bis[1-(biphenyl-2-yloxy)propan-2-ol] |
| Edukte: | 14,3 g 2-[(Biphenyl-2-yloxy)methyl]oxiran |
| | 38 mg 1-Butyl-3-Methylimidazoliumbromid |
| | 4,5 g 1,6-Hexandithiol |
| Bedingungen: | Reaktionstemperatur 60 °C beim Zutropfen, 70 °C, Reaktionszeit: 25 h |
| | Es wurde eine klare, farblose, viskose Flüssigkeit erhalten. |
| $^1$H-NMR | ($CDCl_3$, 400 MHz): δ (1H)= 7,45 (d, 2H), 7,38 (t, 2H), 7,32 (m, 3H), 7,16 (t, 1H), 6,97 (d, 1H), 4,05 (d, 2H), 3,95 (m, 1H), 2,65 (dd, 1H), 2,58 (dd, 1H), 2,45-2,52 (m, 3H), 1,5 (m, 2H), 1,35 (m, 2H) |

| | |
|---|---|
| **Beispiel 6a** | 3,3'-[Oxybis(ethan-2,1-diylsulfanediyl)]bis[1-(biphenyl-2-yloxy)propan-2-ol] |
| Edukte: | 14,3 g 2-[(Biphenyl-2-yloxy)methyl]oxiran |
| | 37 mg 1-Butyl-3-Methylimidazoliumbromid |
| | 3,9 g 2,2'-Oxydiethantbiol |
| Bedingungen: | Reaktionstemperatur 60 °C beim Zutropfen, dann 25 h Reaktionszeit bei 70 °C dann 24 h bei 80 °C |
| | Es wurde eine klare, farblose, viskose Flüssigkeit erhalten. |
| $^1$H-NMR | ($CDCl_3$, 400 MHz): $\delta$ (1H)= 7,45 (d, 2H), 7,38 (t, 2H), 7,32 (m, 3H), 7,16 (t, 1H), 6,97 (d, 1H), 3,9 - 4,05 (m, 3H), 3,55 (t, 2H), 2,87 (t, 1H, OH), 2,55-2,85 (m, 4H). |

| | |
|---|---|
| **Beispiel 7a** | 1,16-Bis(biphenyl-2-yloxy)-7,10-dioxa-4,13-dithiahexadecan-2,15-diol |
| Edukte: | 14,3 g 2-[(Biphenyl-2-yloxy)methyl]oxiran |
| | 39 mg 1-Butyl-3-Methylimidazoliumbromid |
| | 5,2 g 1,2-Bis(2-Mercaptoethoxy)ethan |
| Bedingungen: | Reaktionstemperatur 60 °C beim Zutropfen, dann 25 h Reaktionszeit bei 70 °C dann 19,5 h bei 80 °C |
| | Es wurd ein klares, blaßgelbes Produkt erhalten. |
| $^1$H-NMR | ($CDCl_3$, 400 MHz): $\delta$ (1H)= 7,45 (d, 2H), 7,38 (t, 2H), 7,32 (m, 311), 7,16 (t, 1H), 6,97 (d, 1H), 3,9 - 4,0 (m, 3H), 3,55 (m, 4H), 2,88 (m, 1H, OH), 2,55-2,65 (m, 4H). |

| | |
|---|---|
| **Beispiel 8a** | 3,3'-(Decan-1,10-diyldisulfanediyl)bis[1-(biphenyl-2-yloxy)propan-2-ol] |
| Edukte: | 14,3 g 2-[(Biphenyl-2-yloxy)methyl]oxiran |
| | 40 mg 1-Butyl-3-Methylimidazoliumbromid |
| | 5,9 g 1,10-Decandithiol |
| Bedingungen: | Reaktionstemperatur 60 °C beim Zutropfen, dann 20 h Reaktionszeit bei 60 °C dann 20,5 h bei 80 °C |
| | Es wurde ein Klares, blaßgelbes Produkt erhalten. |
| $^1$H-NMR | ($CDCl_3$, 400 MHz): $\delta$ (1H)= 7,45 (d, 2H), 7,38 (t, 2H), 7,32 (m, 3H), 7,16 (t, 1H), 6,97 (d, 1H), 4,03 (d, 2H), 3,98 (m, 1H), 2,68 (dd, 1H, 2,55 (dd, 1H), 2,48 (t+s(b), 3H), 1,50 (p, 2H), 1,35 (m, 2H), 1,25 (s, 4H) |

| | |
|---|---|
| **Beispiel 9a** | Ethan-1,2-diylbis({[3-(biphenyl-2-yloxy)-2-hydroxy-propyl]sulfanyl}acetat) |
| Edukte: | 14,3 g 2-[(Biphenyl-2-yloxy)methyl]oxiran |
| | 41 mg 1-Butyl-3-Methylimidazoliumbromid |
| | 6,0 g Glycoldimercaptoacetat |
| Bedingungen: | Reaktionstemperatur 60 °C beim Zutropfen, dann 25,5 h Reaktionszeit bei 70 °C |
| | Es wurde ein klares, gelbes Produkt erhalten. |
| $^1$H-NMR | ($CDCl_3$, 400 MHz): $\delta$ (1H)= 7,45 (d, 2H), 7,38 (t, 2H), 7,32 (m, 3H), 7,16 (t, 1H), 6,97 (d, 1H), 4,28 (s, 2H), 4,00 (m, 3H), 3,23 (s, 2H), 2,6-2,85(m, 3H) |

| | |
|---|---|
| **Beispiel 10a** | 2-{[3-(Biphenyl-2-yloxy)-2-hydroxypropyl]sulfanyl}-3-{[3-(biphenyl-2-yloxy)-2-hydroxypropyl]sulfanyl}propan-1-ol |
| Edukte: | 38,2 g 2-[(Biphenyl-2-yloxy)methyl]oxiran |
| | 96 mg Triphenylphosphin |
| | 9,9g 2,3-Disulfanylpropan-1-ol |
| Bedingungen: | Die Vorlage wurde auf 55 °C eingestellt. Während des Zudosierens des 2,3-Disulfanylpropan-1-ol über 1 Stunde erwärmte sich die Reaktionsmischung auf 70 °C. Die Reaktion war exotherm (Kühlung!). Anschließend wurde 24 h bei 60 °C weitergerührt. |
| | Es wurde ein klares, farbloses, hochviskoses Produkt erhalten. |

(fortgesetzt)

| | |
|---|---|
| $^1$H-NMR | ($CDCl_3$, 400 MHz): δ (1H)= 7,46 (d, 4H), 7,38 (t, 4H), 7,32 (m, 6H), 7,16 (t, 2H), 6,98 (dd, 2H), 3,93-4,02 (m, 6H), 3,57 (m, 1H), 3,40 (m, 1H), 2,88 (m,1H), 2,53-2,75 (m, 6H), 2,50 (m, 1H), 2,28 (m, 1H). |

| | |
|---|---|
| **Beispiel 11a** | 3,3'-[Sulfanediylbis(benzol-4,1-diylsulfanediyl)]bis[1-(biphenyl-2-yloxy)propan-2-ol] |
| Edukte: | 9,6 g 2-[(Biphenyl-2-yloxy)methyl]oxiran |
| | 13 mg Triphenylphosphin |
| | 6,0 g 4,4'-Sulfanediyldibenzolthiol |
| Bedingungen; | Reaktionstemperatur 60 °C, Reaktionszeit bei 30,5 h |
| | Es wurde ein klares, glasiges Produkt erhalten. |
| $^1$H-NMR | ($CDCl_3$, 400 MHz): δ (1H)= 7,45 (d, 2H), 7,38 (t, 2H), 7,30 (m, 3H), 7,18 (m, 4H), 7,07 (t, 1H), 6,92 (d, 1H), 4,04 (m, 2H), 3,96 (m, 1H), 3,09 (dd, 1H), 2,96 (dd, 1H), 2,39 (d, 1H, OH) |

**Vorstufe zu Beispiel 12a** 2-[(biphenyl-3-yloxy)methyl]oxiran

7,4g 3-Phenylphenol, 52,7g 2-Butanon und 21,5g Kaliumcarbonat wurden in einem Dreihalskolben vorgelegt, der mit KPG-Rührer und Rührmotor sowie Trockenrohr ausgerüstet war. Es wurde auf 22 °C temperierte und 23,8g Epibromhydrin innerhalb von ca. 2 Stunden zugetropfte, die Temperatur auf 80 °C erhöhte und dann weitere 45 Stunden bei ca. 75 °C gerührt. Anschließend wurde abfiltriert und das Lösemittel am Rotationsverdampfer entfernt. Es wurde ein klares, gelbliches, dünnflüssiges Produkt erhalten.

$^1$H-NMR (CDC13,400 MHz): δ (1H)= 7,57 (d, 2H), 7,42 (t, 2H), 7,33 (m, 2H), 7,20 (d, 1H), 7,16 (t, 1,7Hz, 1H), 6,90 (dd, 1H), 4,28 (dd, 1H), 4,02 (dd, 1H), 3,37 (m, 1H), 2,91 (dd, 1H), 2,77 (dd, 1H).

| | |
|---|---|
| **Beispiel 12a** | 3,3'-(Propan-1,3-diyldisulfanediyl)bis[1-(biphenyl-3-yloxy)propan-2-ol] |
| Edukte: | 7,5 g Produkt aus der Vorstufe zu Beispiel 12a |
| | 72 mg 1-Butyl-3-Methylimidazoliumbromid |
| | 1,3g 1,3-Propandithiol |
| Bedingungen: | Reaktionstemperatur 60 °C während des Zudosierens, dann 80 °C; Reaktionszeit bei 47h |
| | Es wurde ein klares, gelbliches, viskoses Produkt erhalten |
| $^1$H-NMR | ($CDCl_3$, 400 MHz): δ (1H)= 7,57 (d, 2H), 7,42 (t, 2H), 7,33 (t, 2H), 7,19 (d, 1H), 7,13 (t, 1,7Hz, 1H), 6,88 (dd, 1H), 4,08-4,15 (m, 3H), 2,87 (dd, 1H), 2,75 (dd, 1H), darunter (b, 1H, OH), 2,69 (t, 2H), 1,90 (p, 1H) |

**Allgemeine Vorschrift zur Herstellung der erfindungsgemäßen Di(meth)acrlyate (Beispiele 1b-12b)**

Die Vorstufe (Beispiel la-12a), Dibutylzinndilaurat und 2,6-Di-tert.-butyl-4-methylphenol wurden in einem Dreihalskolben vorgelegt, der mit KPG-Rührer und Rührmotor, Gaseinlass sowie Trockenrohr ausgerüstet war. Es wurde auf 60 °C temperiert, langsam Luft übergeleitet und das 2-Isocyanatoethylacrylat innerhalb ca. einer halben Stunde zugetropft. Es wurde weiter gerührt bis im IR-Spektrum keine NCO-Bande (2261cm$^{-1}$) mehr beobachtet werden konnte.

| | |
|---|---|
| **Beispiel 1b** | 6,13-Bis[(biphenyl-2-yloxy)methyl]-4,15,20-trioxo-5,14,19-trioxa-8,11-dithia-3,16-diazadocos-21-en-1-ylacrylat |
| Edukte: | 17,2g des Produktes aus Beispiel 1a |
| | 8,5g 2-Isocyanatoethylacrylat |
| | 13mg Dibutylzinndilaurat |
| | 3mg 2,6-Di-tert.-butyl-4-methylphenol |
| Bedingungen: | Reaktionszeit 19h bei 60 °C |
| | Es wurde ein klares, farbloses, hochviskoses Produkt erhalten. |
| $n_D^{20}$ | 1,5904 (589nm) |

| | |
|---|---|
| **Beispiel 2b** | 6,14-Bis[(biphenyl-2-yloxy)methyl]-4,16,21-trioxo-5,15,20-trioxa-8,12-dithia-3,17-diazatricos-22-en-1-ylacrylat |
| Edukte: | 17,6 g Produkt aus Beispiel 2a |
| | 8,5 g 2-Isocyanatoethylacrylat |
| | 13 mg Dibutylzinndilaurat |
| | 3 mg 2,6-Di-tert.-butyl-4-methylphenol |
| Bedingungen: | Reaktionszeit 3h 50 Minuten bei 60 °C, nach 3 Tagen bei Raumtemperatur Lagerung kein NCO im IR nachweisbar. |
| | Es wurde ein klares, farbloses, hochviskoses Produkt erhalten. |
| $n_D^{20}$ | 1,5864 (589nm) |

| | |
|---|---|
| **Beispiel 3b** | 6,15-Bis[(biphenyl-2-yloxy)methyl]-4,17,22-trioxo-5,16,21-trioxa-8,13-dithia-3,18-diazatetracos-23-en-1-ylacrylat |
| Edukte: | 21,0 g Produkt aus Beispiel 3a |
| | 9,9 g 2-Isocyanatoethylacrylat |
| | 15 mg Dibutylzinndilaurat |
| | 3 mg 2,6-Di-tert.-butyl-4-methylphenol |
| Bedingungen: | Zutropfen (exotherm!) in 15 Minuten bei 60 °C, |
| | dann Reaktionszeit von 18,7h bei 60 °C |
| | Es wurde ein klares, fast farbloses, hochviskoses Produkt erhalten. |
| $n_D^{20}$ | 1,5846 (589nm) |

| | |
|---|---|
| **Beispiel 4b** | 6,13-Bis[(biphenyl-2-yloxy)methyl]-9,10-dimethyl-4,15,20-trioxo-5,14,19-trioxa-8,11-dithia-3,16-diazadocos-21-en-1-ylacrylat |
| Edukte: | 18,0 g Produkt aus Beispiel 4a |
| | 8,5 g 2-Isocyanatoethylacrylat |
| | 13 mg Dibutylzinndilaurat |
| | 3 mg 2,6-Di-tert.-butyl-4-methylphenol |
| Bedingungen: | Zutropfen (exotherm!) in 35 Minuten bei 60 °C, |
| | dann Reaktionszeit von 16 h bei 60 °C |
| | Es wurde ein klares, fast farbloses, hochviskoses Produkt erhalten. |
| $n_D^{20}$ | 1,5840 (589nm) |

| | |
|---|---|
| **Beispiel 5b** | 6,17-Bis[(biphenyl-2-yloxy)methyl]-4,19,24-trioxo-5,18,23-trioxa-8,15-dithia-3,20-diazahexacos-25-en-1-ylacrylat |
| Edukte: | 18,9 g Produkt aus Beispiel 5a |
| | 8,5 g 2-Isocyanatoethylacrylat |
| | 14 mg Dibutylzinndilaurat |
| | 3 mg 2,6-Di-tert.-butyl-4-methylphenol |
| Bedingungen: | Zutropfen (exotherm!) in 35 Minuten bei 60 °C, |
| | dann Reaktionszeit von 16 h bei 60 °C |
| | Es wurde ein klares, fast farbloses, hochviskoses Produkt erhalten. |
| $n_D^{20}$ | 1,5801 (589nm) |

| | |
|---|---|
| **Beispiel 6b** | 6,16-Bis[(biphenyl-2-yloxy)methyl]-4,18,23-trioxo-5,11,17,22-tetraoxa-8,14-dithia-3,19-diazapentacos-24-en-1-ylacrylat |
| Edukte: | 18,2 g Produkt aus Beispiel 6a |
| | 8,5 g 2-Isocyanatoethylacrylat |
| | 13 mg Dibutylzinndilaurat |
| | 5 mg 2,6-Di-tert.-butyl-4-methylphenol |
| Bedingungen: | Reaktionszeit 4 h |
| | Es wurde ein klares, blaßgelbes, hochviskoses Produkt erhalten. |
| $n_D^{20}$ | 1,5814 (589nm) |

| | |
|---|---|
| **Beispiel 7b** | 6,19-Bis[(biphenyl-2-yloxy)methyl]-4,21,26-trioxo-5,11,14,20,25-pentaoxa-8,17-dithia-3,22-diazaoctacos-27-en-1-ylacrylat |
| Edukte: | 19,5 g Produkt aus Beispiel 7a |
| | 8,5 g 2-Isocyanatoethylacrylat |
| | 14 mg Dibutylzinndilaurat |
| | 6 mg 2,6-Di-tert.-butyl-4-methylphenol |
| Bedingungen: | Reaktionszeit 4 h |
| | Es wurde ein klares, blaßgelbes, hochviskoses Produkt erhalten. |
| $n_D^{20}$ | 1,5775 (589nm) |

| | |
|---|---|
| **Beispiel 8b** | 6,21-Bis[(biphenyl-2-yloxy)methyl]-4,23,28-trioxo-5,22,27-trioxa-8,19-dithia-3,24-diazatriacont-29-en-1-ylacrylat |
| Edukte: | 20,2 g Produkt aus Beispiel 8a |
| | 8,5 g 2-Isocyanatoethylacrylat |
| | 14 mg Dibutylzinndilaurat |
| | 6 mg 2,6-Di-tert.-butyl-4-methylphenol |
| Bedingungen: | Reaktionszeit 24 h |
| | Es wurde ein klares, blaßgelbes, hochviskoses Produkt erhalten. |
| $n_D^{20}$ | 1,5702 (589nm) |

| | |
|---|---|
| **Beispiel 9b** | 8-[(Biphenyl-2-yloxy)methyl]-4,10,15-trioxo-3,9,14-trioxa-6-thia-11-azaheptadec-16-en-1-yl-(5S)-5-[(biphenyl-2-yloxy)ethyl]-7,12-dioxo-6,11-dioxa-3-thia-8-azatetradec-13-en-1-oat |
| Edukte: | 20,3 g Produkt aus Beispiel 9a |
| | 8,5 g 2-Isocyanatoethylacrylat |
| | 14 mg Dibutylzinndilaurat |
| | 6 mg 2,6-Di-tert.-butyl-4-methylphenol |
| Bedingungen: | Reaktionszeit 18 h |
| | Es wurde ein klares, gelbes, hochviskoses Produkt erhalten. |
| $n_D^{20}$ | 1,5768 (589nm) |

| | |
|---|---|
| **Beispiel 10b** | 6,13-Bis[(biphenyl-2-yloxy)methyl]-4,15,20-trioxo-10-[({[2-(phenylsulfanyl)phenyl]carbamoyl}oxy)methyl]-5,14,19-trioxa-8,11-dithia-3,16-diazadocos-21-en-1-ylacrylat |
| Edukte: | 4,8 g Produkt aus Beispiel 10a (Triol) |
| | 1,8 g 1-Isocyanato-2-(phenylsulfanyl)benzol |

(fortgesetzt)

| | |
|---|---|
| | 2,3 g 2-Isocyanatoethylacrylat |
| | 4 mg Dibutylzinndilaurat |
| | 1 mg 2,6-Di-tert.-butyl-4-methylphenol |
| | 20g Chloroform |
| Bedingungen: | Zunächst wurden Triol, Lösemittel, Dibutylzinndilaurat und 2,6-Di-tert.-butyl-4-methylphenol bei 30 °C vorgelegt und das 1-Isocyanato-2-(phenylsulfanyl)benzol innerhalb von 7 Minuten zugetropft. Es wurde für 2 h 45 Min weitergerührt. Als im IR-Spektrum das NCO-Signal weitgehend verschwunden war, wurde innerhalb von 70 Minuten langsam auf 60 °C erwärmt und das 2-Isocyanatoethylacrylat innerhalb von 5 Minuten zugetropft. Es wurde für weitere 14,5 h gerührt bis kein NCO-Signal mehr im IR-Spektrum erkennbar war. Dann wurde das Lösemittel am Rotationsverdampfer entfernt. |
| | Es wurde ein klares, hochviskoses Produkt erhalten |
| $n_D^{20}$ | 1,6002 (589nm) |

| | |
|---|---|
| **Beispiel 11b** | Sulfanediylbis{benzol-4,1-diylsulfanediyl[3-(biphenyl-2-yloxy)propan-1,2-diyl]oxycarbonyliminoethan-2,1-diyl}bisacrylat |
| Edukte: | 14,6 g Produkt aus Beispiel 11 a |
| | 5,6 g 2-Isocyanatoethylacrylat |
| | 10 mg Dibutylzinndilaurat |
| | 2 mg 2,6-Di-tert.-butyl-4-methylphenol |
| Bedingungen: | Reaktionszeit 23 h. |
| | Es wurde eine klares, glasiges Produkt erhalten. |
| $n_D^{20}$ | 1,6300 (589nm) |
| $^1$H-NMR | ($CDCl_3$, 400 MHz): δ (1H)= 7,47 (d, 2H), 7,23-7,37 (m, 6H), 7,18 (m, 4H, AA'BB'), 7,04 (t, 1H), 6,92 (d, 1H), 6,41 (d, 1H), 6,10 (dd, 1H), 5,83 (dd, 1H), 5,08 (p, 1H), 4, 87 (t, 1H, NH), 4,10-4,23 (m, 4H), 3,43 (q, 2H), 3,12 (d, 2H) |

| | |
|---|---|
| **Beispiel 12b** | 6,14-Bis[(biphenyl-3-yloxy)methyl]-4,16,21-trioxo-5,15,20-trioxa-8,12-dithia-3,17-diazatricos-22-en-1-ylacrylat |
| Edukte: | 9,3 g Produkt aus Beispiel 12a |
| | 4,6 g 2-Isocyanatoethylacrylat |
| | 7 mg Dibutylzinndilaurat |
| | 1 mg 2,6-Di-tert.-butyl-4-methylphenol |
| Bedingungen: | Reaktionszeit 1h 50 Min. |
| | Es wurde ein klares, gelbliches, hochviskoses Produkt erhalten. |
| $n_D^{20}$ | 1,5908 (589nm) |

**Vergleichsbeispiel 1**: Phosphorothioyltris(oxybenzol-4,1-diylcarbamoyloxyethan-2,1-diyl)trisacrylat [1072455-04-9]

In einem 500 mL Rundkolben wurden 0.1 g 2,6-Di-tert.-butyl-4-methylphenol, 0.05 g Dibutylzinndilaurat sowie und 213.07 g einer 27 %-igen Lösung von Tris(p-isocyanatophenyl)thiophosphat in Ethylacetat vorgelegt und auf 60 °C erwärmt. Anschließend wurden 42.37 g 2-Hydroxyethylacrylat zugetropft und die Mischung weiter auf 60 °C gehalten, bis der Isocyanatgehalt unter 0.1 % gesunken war. Danach wurde abgekühlt und im Vakuum das Ethylacetat vollständig entfernt. Das Produkt wurde als teilkristalliner Feststoff erhalten. Das erhaltene Produkt hat einen $n^D{}_{20}$= 1,5430 (589nm).

**Vergleichsbeispiel 2:** Benzol-1,3-diylbis[oxy-3-(biphenyl-4-yloxy)propan-1,2-diyl]bisacrylat **Vergleichsbeispiel 2 - 1. Stufe:** Umsetzung von 2,2'-[Benzol-1,3-diylbis(oxymethylen)]-dioxiran (Resorcindiglycidylether) zu 1,1'-[Benzol-1,3-diylbis(oxy)]bis[3-(biphenyl-4-yloxy)propan-2-ol]

In Analogie zu Beispiel 1 [0192] in EP 1 627 867 A1 (Umsetzung von Formel 10-1 zu Formel 5-1) wurde 18,7 g 4-Phenylphenol und 1,3 mg Triphenylphosphin bei 160 °C aufgeschmolzen. Nun gab man 11,8 g Resorcindiglycidylether (kommerziell erhältlich als Denacol LSC 201 von Nagase & Co., Ltd. Japan) hinzu und rührt zehn Stunden lang. Nun gab man weitere 3,9 mg Triphenylphosphin hinzu und rührte bei 170 °C weitere 24 h lang. Nach dem Erkalten wurde das Produkt in Butylacetat umkristallisiert. $^1$H-NMR entspricht: $^1$H-NMR (400MHz, CDC13): 2,54 (d, 2H, OH), 4,10-4,22 (m, 8H, OCH2CH), 4,35-4,45 (m, 2H, CH2CHOH-$CH_2$), 6,5-6,6 (m, 3H), 7,0 (AA'BB', 4H), 7,20 (t, 1H), 7,30 (t, 2H), 7,40 (t, 4H), 7,50-7,55 (m, 8H)

**Vergleichsbeispiel 2 - 2. Stufe:** Umsetzung der 1. Stufe zu Benzol-1,3-diylbis[oxy-3-(biphenyl-4-yloxy)propan-1,2-diyl]bis(3-chlorpropanoat)

In Analogie zu Beispiel 2 [0195] in EP 1 627 867 A1 (Umsetzung Formel 5-1 zu Formel 6-1) wurden 2,9 g des Vergleichsbeispiels 2 - 1. Stufe in 50g Butylacetat bei 68 °C erwärmt. Man gab 1,8 g Triethylamin hinzu und erwärmte auf 85°C, wo man eine klare Lösung erhielt. Nun tropfte man 2,3 g 3- Chlorpropionsäurechlorid innerhalb einer halben Stunde hinzu. Die Lösung erwärmte sich dabei auf bis zu 126 °C. Man temperierte auf 112 °C und rührte für weitere vier Stunden und kühlte ab. Das Rohprodukt wurde in 200 ml Eiswasser gegeben und mit 150 ml Toluol extrahiert. Nach dem Trennen der Phasen wurde die Toluolphase mit 100 ml 3%iger $NaHCO_3$-Lösung gewaschen. Nach Abdestillation der Toluols im Rotationsverdampfer bei 40°C erhielt man das Rohprodukt. Das $^1$H-NMR entspracht, zeigte zudem Mengen des gewünschten Endproduktes (Eliminierungsprodukts).

**Vergleichsbeispiel 2 - 3. Stufe:** Umsetzung der 2. Stufe zu Benzol-1,3-diylbis[oxy-3-(biphenyl-4-yloxy)propan-1,2-diyl]bisacrylat

Gemäß Beispiel 1 [0128] in EP 1 627 867 A1 (Eliminierung zu Formel 1-1) wurden 2,77 g des Rohprodukts in 50 g Aceton gelöst und auf 5 °C temperiert. Nun gab man 1,25 g Triethylamin innerhalb von 30 Minuten zu und hielt die Lösung für 70 Stunden bei 5 °C. Man erwärmte auf 25 °C und gab 100 g Toluol und 100 g Wasser hinzu. Die Phasen wurden getrennt und die Toluolphase mit 10 ml 5%-ige Salzsäure gewaschen. Anschließend wurde achtmal mit 50 ml Wasser bis das Waschwasser neutral war weiter gewaschen. 0,2 mg p-Methoxyphenol wurden zugegeben und am Rotationsverdampfer wurde das Produkt bei 40°C vom Lösemittel weitgehend befreit. $^1$H-NMR (400MHz, CDC13): 4,10-4,40 (m, 8H), 5,5 (m, 2H, CH), 5,85 (d, 2H, Acrylat), 6,18 (dd, 2H, Acrylat), 6,45 (d, 2H, Acrylat), 6,50-6,60 (m, 3H), 6,98 (AA'BB', 4H), 7,20 (t, 1H), 7,30 (t, 2H), 7,38 (t, 4H), 7,50 (m, 8H).

**Polyol-Komponente:**

In einem 1 L Kolben wurden 0.18 g Addocat® SO, 374.8 g ε-Caprolacton und 374.8 g eines difunktionellen Polytetrahydrofuranpolyetherpolyols (Equivalentgewicht 650 g/Mol OH) vorgelegt und auf 120 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsiger Feststoff erhalten.

Urethanacrylat 1: 2-({[3-(Methylsulfanyl)pharyl]carbamoyl}oxy)ethylprop-2-enoat

In einem 100 mL Rundkolben wurden 0,02 g 2,6-Di-tert.-butyl-4-methylphenol, 0,01 g Desmorapid Z, 11,7 g 3-(Methylthio)phenylisocyanat vorgelegt und auf 60 °C erwärmt. Anschließend wurden 8,2 g 2-Hydroxyethylacrylat zugetropft und die Mischung weiter auf 60 °C. gehalten, bis der Isocyanatgehalt unter 0,1 % gesunken war. Danach wurde abgekühlt. Das Produkt wurde als farblose Flüssigkeit erhalten.

**Fluoriertes Urethan:** Bis(2,2,3,3,4,4,5,5,6,6,7,7-dodecafluoroheptyl)-(2,2,4-trimethylhexane-1,6-diyl)biscarbamat

In einem 6L-Rundkolben wurden 0,50 g Desmorapid Z und 1200 g Trimethylhexamethylendiisocyanat vorgelegt und auf 80 °C erwärmt. Anschließend wurden 3798 g 1H,1H,7H-Perfluorheptan-1-ol zugetropft und die Mischung weiter auf 80 °C gehalten, bis der Isocyanatgehalt unter 0,1 % gesunken war. Danach wurde abgekühlt. Das Produkt wurde als farbloses Öl erhalten.

**Herstellung der erfindungsgemäßen und nicht erfindungsgemäßen Medien (Coupons) (Beispiele 13 bis 24 und Vergleichbeispiele 3 und 4)**

2,940 g der oben beschrieben Polyol-Komponente wurden mit 2.000 g des jeweiligen Acrylats (Beispiele 1 bis 12, Vergleichbeispiel 1), 2,000 g des oben beschriebenen Urethanacrylats 1, 2,000 g des oben beschriebenen fluorierten Urethans, 0,15 g CGI 909, 15 mg Kristallviolett, 15 mg Irgacure 250, 15 mg Glasperlen der Größe 9,18 μm und 0,517 g g N-Ethylpyrolidon bei 60 °C gemischt, so dass eine klare (in manchen Fällen leicht trübe) Lösung erhalten wurde. Anschließend wurde auf 30 °C abgekühlt, 545 mg Desmodur® N 3900 zugegeben und erneut gemischt. Schließlich wurden 6 mg Fomrez UL 28 zugegeben und erneut kurz gemischt. Die erhaltene flüssige Masse wurde dann auf eine Glasplatte (Fa. Corning, NY 14831, USA, Micro slide plane: Dicke 0,96-1,06mm, 75mm x 50mm, Type: 2947-75x50) gegeben und dort mit einer zweiten Glasplatte abgedeckt. Dieser Probenkörper wurde 12 Stunden bei Raumtemperatur gelagert und dabei ausgehärtet. Anschließend wurden die Medien lichtdicht verpackt.

**Bestimmung der physikalischen Daten der erfindungsgemäßen und nicht erfindungsgemäßen Medien**

Die Messung der holographischen Eigenschaften DE und Δn wurde gemäß dem oben im Abschnitt "Messmethoden" beschriebenen Verfahren durchgeführt.

Die Trübungsmessung wurde ebenfalls gemäß dem oben im Abschnitt "Messmethoden" beschriebenen Verfahren durchgeführt, wobei vor der Messung das jeweilige Medium zunächst bei Raumtemperatur und Umgebungslicht ca. 15-30 Minuten gebleicht wurde, bis die Farbe visuell nicht mehr zu erkennen war.

Die Ergebnisse der Messungen sind in der nachfolgenden Tabelle dargestellt:

| | | | Anzahl der Atome in R | | | | | Brechungsindex | Haze | Brechungsindexkontrast (Δn) |
|---|---|---|---|---|---|---|---|---|---|---|
| Beispiel | Acrylat | R des erfindungsgemäßen Di(meth)acrylat | Kohlenstoff | Sauerstoff | Schwefel | Stickstoff | Summe | (bei 589nm) | [%] | bei 15,9 [mJ/cm2] |
| 13 | 7b | HS-CH2-CH2-O-CH2-CH2-O-CH2-CH2-SH | 6 | 2 | 2 | - | 10 | 1,5775 | 0,9 | 0,0290 |
| 14 | 4b | HS-CH(CH3)-CH(CH3)-SH | 4 | - | 2 | - | 6 | 1,5840 | 1,0 | 0,0315 |
| 15 | 6b | HS-CH2-CH2-O-CH2-CH2-SH | 4 | 1 | 2 | - | 7 | 1,5814 | 1,4 | 0,0292 |
| 16 | 10b | HS-CH2-CHR1-SH, R1= CH2O-CO-NH-PhSPh | 16 | 2 | 3 | 1 | 22 | 1,6002 | 3,5 | 0,0280 |
| 17 | 8b | HS-[CH2]10-SH | 10 | - | 2 | - | 12 | 1,5702 | 33,4 | 0,0306 |
| 18 | 11b | HS-Ph-S-Ph-SH | 12 | - | 3 | - | 15 | 1,6300 | 36,0 | 0,0260 |
| 19 | 5b | HS-CH2-CH2-CH2-CH2-CH2-CH2-SH | 6 | - | 2 | - | 8 | 1,5801 | 36,2 | 0,0295 |
| 20 | 3b | HS-CH2-CH2-CH2-CH2-SH | 4 | - | 2 | - | 6 | 1,5846 | 39,5 | 0,0252 |
| 21 | 9b | HS-CH2-CO-O-CH2-CH2-O-CO-CH2-SH | 6 | 4 | 2 | - | 12 | 1,5768 | 40,7 | 0,0322 |
| 22 | 12b | HS-CH2-CH2-CH2-SH* | 3 | - | 2 | - | 5 | 1,5908 | 41,3 | 0,0345 |
| 23 | 2b | HS-CH2-CH2-CH2-SH | 3 | - | 2 | - | 5 | 1,5864 | 55,6 | 0,0295 |
| 24 | 1b | HS-CH2-CH2-SH | 2 | - | 2 | - | 4 | 1,5904 | 61,7 | 0,0273 |
| | | wenn nicht anders beschrieben wurde 2-[(Biphenyl-2-yloxy)methyl]oxiran verwendet | | | | | | | | |
| | | * 2-[(biphenyl-3-yloxy)methyl]oxiran wurde verwendet | | | | | | | | |
| | | Ph = Phenyl bzw. Phenylen | | | | | | | | |
| Vergleichsbeispiel 3 | Vergleichsbeispiel 1 | Phosphorothioyltris(oxybenzol-4,1-diylcarbamoyloxyethan-2,1-diyl)trisacrylat | | | | | | 1,5430 | 54,9 | 0,0240 |
| Vergleichsbeispiel | Vergleichsbeispiel | Benzol-1,3-diylbis[oxy-3-(biphenyl-4-yloxy)propan-1,2- | | | | | | 1,6025* | 45,0 | 0,0240 |

| | | | | | | | | | | |
|---|---|---|---|---|---|---|---|---|---|---|
| spiel 4 | spiel 2 | diyl]bisacrylat | | | | | | | | |

*Vergleichsbeispiel 2 ist ein kristalline Verbindung. Der Brechungsindex wurde daher nach Aufschmelzen der Probe und durch zügigem Transfer ins Refraktometer bestimmt.

Der Meßwert wurde kurz vor dem Rekristallisieren im Refraktometer bestimmt.

Wie aus der Tabelle hervorgeht, zeigen die Medien, die eine erfindungsgemäße Verbindung gemäß Formel (I) als Schreibmonomer enthalten, einen deutlich höheren Brechungsindexkontrast $\Delta n$, als das Medium des Vergleichsbeispiels 3, dass wiederum ein aus der WO 2008/125199 bekanntes Schreibmonomer beinhaltet. Dies gilt ebenso für das Medium des Vergleichsbeispiels 4, bei dem ein aus der EP 1 627 867 A1 bekanntes Schreibmonomer verwendet wurde. Darüber hinaus zeichnen sich die mit Hilfe der erfindungsgemäßen Verbindungen hergestellten Medien aber auch durch eine besonders geringe Trübung (Haze) auf, was für optische Artikel im allgemeinen und für Transmissions-Hologramme im besonderen vorteilhaft ist.

## Patentansprüche

**1.** Verbindung der Formel (I)

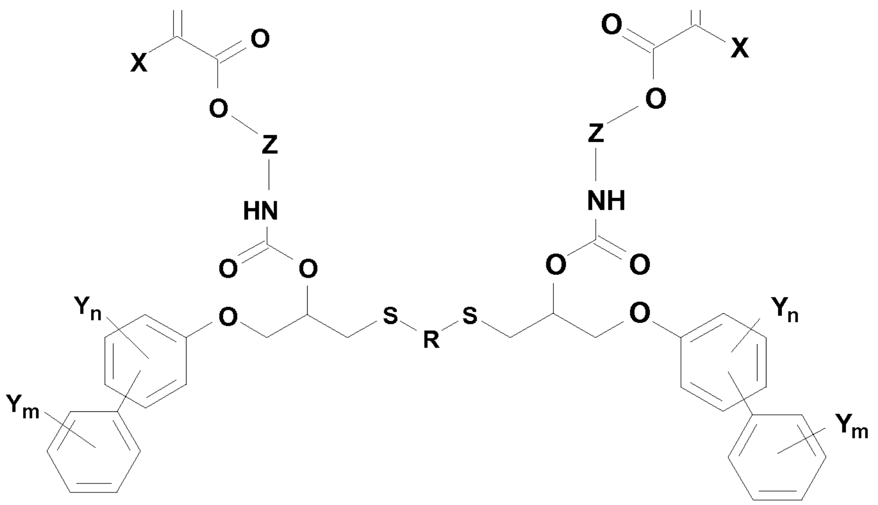

Formel (I)

in der

X $CH_3$ oder Wasserstoff,
Z ein linearer oder verzweigter C2 bis C4-Alkylrest,
R ein linearer oder verzweigter, gegebenenfalls mit Heteroatomen substituierter aliphatischer, aromatischer oder araliphatischer Rest, oder ein linearer oder verzweigter, gegebenenfalls mit Heteroatomen substituierter aliphatischer, aromatischer oder araliphatischer Ether (-O-), Thioether (-S-), Ester (-O-CO) oder Urethan (-NH-(C=O)-O-),
Y jeweils unabhängig voneinander Wasserstoff, Methyl, Ethyl, Propyl, n-Butyl, tert.-Butyl, Chlor, Brom, Iod, Methylthio, Phenyl oder Phenylthio,
n 0 bis 4 und
m 0 bis 5

sind.

**2.** Verbindung nach Anspruch 1, **dadurch gekennzeichnet, dass** R ein linear oder verzweigte aliphatischer, aromatischer oder araliphatischer Rest mit 2 bis 22 Kohlenstoff-Atomen ist, der bevorzugt mit einem oder mehreren Sauerstoff-, Stickstoff- und/oder Schwefel-Atomen substituiert ist.

**3.** Verbindung nach Anspruch 2, **dadurch gekennzeichnet, dass** R 2 bis 16 Kohlenstoff-, 0 bis 4 Sauerstoff-, 0 bis 1 Stickstoff- und 0 bis 1 Schwefel-Atome aufweist.

**4.** Verbindung nach einem der Ansprüche 1 bis 3, **dadurch gekennzeichnet, dass** R wenigstens eine funktionelle Gruppe ausgewählt aus der Gruppe Ether (-O-), Thioether (-S-), Ester (-O-CO), Urethan (-NH-(C=O)-O-)umfasst.

5. Verbindung nach einem der Ansprüche 1 bis 4, **dadurch gekennzeichnet, dass** X Wasserstoff ist.

6. Verbindung nach einem der Ansprüche 1 bis 5, **dadurch gekennzeichnet, dass** Y jeweils unabhängig voneinander H, Methyl, Phenyl, Methylthio oder Phenylthio und bevorzugt Wasserstoff sind.

7. Verbindung nach einem der Ansprüche 1 bis 6, **dadurch gekennzeichnet, dass** $n \geq 1$ und $\leq 4$ und/oder $m \geq 1$ und $\leq 5$ und bevorzugt $m + n < 4$ sind.

8. Verbindung nach einem der Ansprüche 1 bis 7, **dadurch gekennzeichnet, dass** Z jeweils ein $(CH_2)_i$-Rest mit $i \geq 2$ und $\leq 4$ oder ein -CH2-CH($CH_3$) -Rest sind.

9. Verwendung einer Verbindung gemäß einem der Ansprüche 1 bis 8 als Schreibmonomer in einer Photopolymer-Formulierung.

10. Photopolymer-Formulierung umfassend wenigstens eine Polyisocyanat-Komponente a), eine Polyol-Komponente b), wobei es sich bei einer Polyol-Komponente um eine polyfunktionelle, isocyanatreaktive Verbindung handelt, die im Mittel wenigstens 1,5 isocyanatreaktive Gruppen pro Molekül aufweist, wobei isocyanatreaktive Gruppen bevorzugt Hydroxy-, Amino- oder Thiogruppen sind, einen Photoinitiator c) und ein Schreibmonomer d), **dadurch gekennzeichnet, dass** wenigstens eine Verbindung gemäß einem der Ansprüche 1 bis 8 als Schreibmonomer d) enthalten ist.

11. Photopolymer-Formulierung nach Anspruch 10, **dadurch gekennzeichnet, dass** als weiteres Schreibmonomer wenigstens ein monofunktionelles Urethan(meth)acrylat enthalten ist.

12. Photopolymer-Formulierung nach einem der Ansprüche 10 oder 11, **dadurch gekennzeichnet, dass** die Polyolkomponente ein difunktioneller Polyether-, Polyester oder ein Polyetherpolyester-block-copolyester mit primären OH-Funktionen ist.

13. Photopolymer-Formulierung nach einem der Ansprüche 10 bis 12, **dadurch gekennzeichnet, dass** die Polyisocyanat-Komponente ein aliphatisches Polyisocyanat oder ein Präpolymer mit primären NCO-Gruppen ist.

14. Photopolymer-Formulierung nach einem der Ansprüche 10 bis 13, **dadurch gekennzeichnet, dass** der Photoinitiator aus einer Kombination von Farbstoffen, deren Absorptionsspektren zumindest teilweise den Spektralbereich von 400 bis 800 nm abdecken, mit wenigstens einem geeigneten Coinitiator besteht.

15. Photopolymer-Formulierung nach einem der Ansprüche 10 bis 14, **dadurch gekennzeichnet, dass** sie zusätzlich Weichmacher, bevorzugt Weichmacher gemäß der allgemeinen Formel (II)

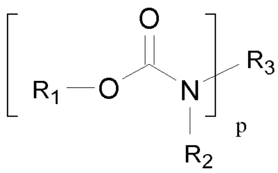

(II)

umfasst, in der $p \geq 1$ und $\leq 8$ ist und $R^1$, $R^2$, $R^3$ Wasserstoff und / oder unabhängig voneinander lineare, verzweigte, cyclische oder heterocyclische unsubstituierte oder gegebenenfalls auch mit Heteroatomen substituierte organische Reste sind, wobei bevorzugt mindestens einer der Reste $R^1$, $R^2$, $R^3$ mit wenigstens einem Fluoratom substituiert ist und besonders bevorzugt $R^1$ ein organischer Rest mit mindestens einem Fluoratom ist.

16. Verwendung einer Photopolymer-Formulierung nach einem der Ansprüche 10 bis 15 zur Herstellung holographischer Medien insbesondere zur Aufzeichnung von In-Line-, Off-Axis-, Full-Aperture-, Transfer-, Weißlicht-, Transmissions-, Denisyuk-, Off-Axis Reflektions- oder Edge-Lit Hologrammen sowie holographischen Stereogrammen.

## Claims

1. Compound of formula (I)

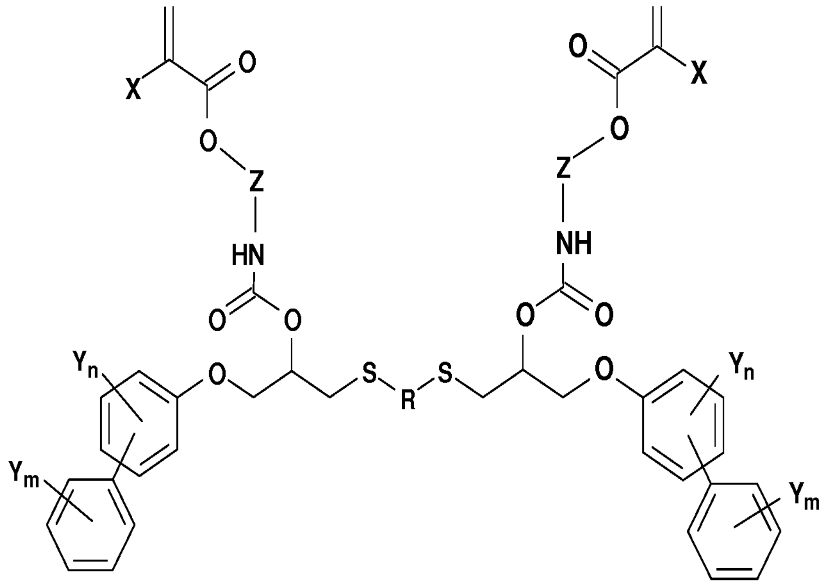

Formula (I)

where

X is $CH_3$ or hydrogen,
Z is a linear or branched C2 to C4 alkyl radical,
R is a linear or branched, optionally heteroatom-substituted aliphatic, aromatic or araliphatic radical, or a linear or branched, optionally heteroatom-substituted aliphatic, aromatic or araliphatic ether (-O-), thioether (-S-), ester (-O-CO) or urethane (-NH-(C=O)-O-),
Y in each occurrence is independently hydrogen, methyl, ethyl, propyl, n-butyl, tert-butyl, chlorine, bromine, iodine, methylthio, phenyl or phenylthio,
n is from 0 to 4 and
m is from 0 to 5.

2. Compound according to Claim 1, **characterized in that** R is a linearly or branched aliphatic, aromatic or araliphatic radical of 2 to 22 carbon atoms, which preferably is substituted with one or more oxygen, nitrogen and/or sulphur atoms.

3. Compound according to Claim 2, **characterized in that** R has 2 to 16 carbon atoms, 0 to 4 oxygen atoms, 0 to 1 nitrogen atoms and 0 to 1 sulphur atoms.

4. Compound according to any one of Claims 1 to 3, **characterized in that** R comprises at least one functional group selected from the group ether (-O-), thioether (-S-), ester (-O-CO), urethane (-NH-(C=O)-O-).

5. Compound according to any one of Claims 1 to 4, **characterized in that** X is hydrogen.

6. Compound according to any one of Claims 1 to 5, **characterized in that** Y in each occurrence is independently H, methyl, phenyl, methylthio or phenylthio and preferably hydrogen.

7. Compound according to any one of Claims 1 to 6, **characterized in that** n is $\geq 1$ and $\leq 4$ and/or m is $\geq 1$ and $\leq 5$ and preferably m + n is < 4.

8. Compound according to any one of Claims 1 to 7, **characterized in that** Z in each occurence is a $(CH_2)_i$ radical with i $\geq 2$ and $\leq 4$ or a -CH2-CH($CH_3$) - radical.

9. Use of a compound according to any one of Claims 1 to 8 as writing monomer in a photopolymer formulation.

10. Photopolymer formulation comprising at least a polyisocyanate component a), a polyol component b), wherein one polyol component is a polyfunctional, isocyanate-reactive compound which has on average at least 1.5 isocyanate-reactive groups per molecule, wherein the isocyanate-reactive groups are preferably hydroxy, amino or thio groups, a photoinitiator c) and a writing monomer d), **characterized in that** at least one compound according to any one of Claims 1 to 8 is present as writing monomer d).

11. Photopolymer formulation according to Claim 10, **characterized in that** at least one monofunctional urethane (meth)acrylate is present as further writing monomer.

12. Photopolymer formulation according to either of Claims 10 and 11, **characterized in that** the polyol component is a difunctional polyether-, polyester or a polyether-polyester block copolyester with primary OH functions.

13. Photopolymer formulation according to any one of Claims 10 to 12, **characterized in that** the polyisocyanate component is an aliphatic polyisocyanate or a prepolymer with primary NCO groups.

14. Photopolymer formulation according to any one of Claims 10 to 13, **characterized in that** the photoinitiator consists of a combination of dyes having absorption spectra covering at least partly the spectral region from 400 to 800 nm, with at least one suitable coinitiator.

15. Photopolymer formulation according to any one of Claims 10 to 14, **characterized in that** it additionally comprises plasticizers, preferably plasticizers according to the general formula (II)

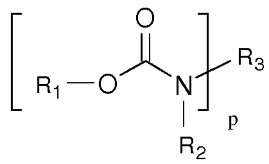

(II)

where p is ≥ 1 and ≥ 8 and $R^1$, $R^2$, $R^3$ are hydrogen and/or independently linear, branched, cyclic or heterocyclic unsubstituted or else optionally heteroatom-substituted organic radicals, wherein preferably at least one of the radicals $R^1$, $R^2$, $R^3$ is substituted with at least a fluorine atom and more preferably $R^1$ is an organic radical having at least one fluorine atom.

16. Use of a photopolymer formulation according to any one of Claims 10 to 15 for producing holographic media especially for recording in-line, off-axis, full-aperture, transfer, white light, transmission, Denisyuk, off-axis reflection or edge-lit holograms and also holographic stereograms.

## Revendications

1. Composé de formule (I)

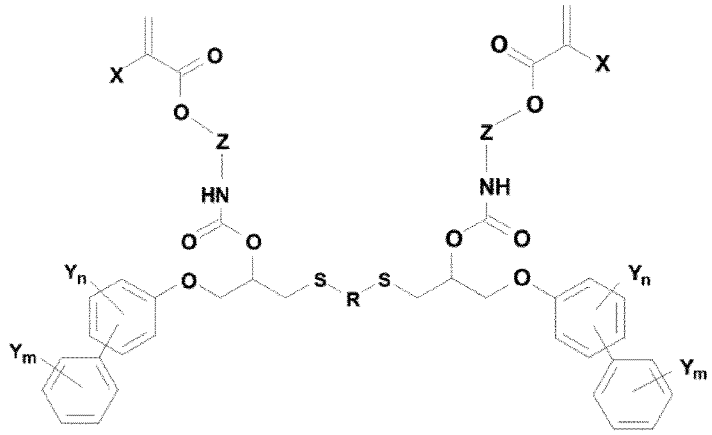

Formule I

dans laquelle

X représente $CH_3$ ou hydrogène,
Z représente un radical alkyle en C2 à C4 linéaire ou ramifié,
R représente un radical aliphatique, aromatique ou araliphatique linéaire ou ramifié, éventuellement substitué avec des hétéroatomes, ou un éther (-O-), thioéther (-S-), ester (-O-CO) ou uréthane (-NH-(C=O)-O-) aliphatique, aromatique ou araliphatique, linéaire ou ramifié, éventuellement substitué avec des hétéroatomes,
les Y représentent chacun indépendamment les uns des autres hydrogène, méthyle, éthyle, propyle, n-butyle, tert.-butyle, chlore, brome, iode, méthylthio, phényle ou phénylthio,
n représente 0 à 4, et
m représente 0 à 5.

2. Composé selon la revendication 1, **caractérisé en ce que** R est un radical aliphatique, aromatique ou araliphatique linéaire ou ramifié de 2 à 22 atomes de carbone, qui est de préférence substitué avec un ou plusieurs atomes d'oxygène, d'azote et/ou de soufre.

3. Composé selon la revendication 2, **caractérisé en ce que** R comprend 2 à 16 atomes de carbone, 0 à 4 atomes d'oxygène, 0 à 1 atome d' azote et 0 à 1 atome de soufre.

4. Procédé selon l'une quelconque des revendications 1 à 3, **caractérisé en ce que** R comprend au moins un groupe fonctionnel choisi dans le groupe constitué par les éthers (-O-), les thioéthers (-S-), les esters (-O-CO), les uréthanes (-NH-(C=O)-O-).

5. Procédé selon l'une quelconque des revendications 1 à 4, **caractérisé en ce que** X représente l'hydrogène.

6. Procédé selon l'une quelconque des revendications 1 à 5, **caractérisé en ce que** les Y représentent chacun indépendamment les uns des autres H, méthyle, phényle, méthylthio ou phénylthio, et de préférence hydrogène.

7. Procédé selon l'une quelconque des revendications 1 à 6, **caractérisé en ce que** $n \geq 1$ et $\leq 4$, et/ou $m \geq 1$ et $\leq 5$, et de préférence $m+n < 4$.

8. Procédé selon l'une quelconque des revendications 1 à 7, **caractérisé en ce que** les Z représentent chacun un radical $(CH_2)_i$ avec $i \geq 2$ et $\leq 4$ ou un radical $-CH_2-CH(CH_3)$.

9. Utilisation d'un composé selon l'une quelconque des revendications 1 à 8 en tant que monomère d'écriture dans une formulation de photopolymère.

10. Formulation de photopolymère comprenant au moins un composant polyisocyanate a), un composant polyol b), un composant polyol étant un composé polyfonctionnel réactif avec les isocyanates, qui comprend en moyenne au

moins 1,5 groupe réactif avec les isocyanates par molécule, les groupes réactifs avec les isocyanates étant de préférence des groupes hydroxy, amino ou thio, un photoinitiateur c) et un monomère d'écriture d), **caractérisée en ce qu'**au moins un composé selon l'une quelconque des revendications 1 à 8 est contenu en tant que monomère d'écriture d).

**11.** Formulation de photopolymère selon la revendication 10, **caractérisée en ce qu'**au moins un (méth)acrylate d'uréthane monofonctionnel est contenu en tant que monomère d'écriture supplémentaire.

**12.** Formulation de photopolymère selon l'une quelconque des revendications 10 et 11, **caractérisée en ce que** le composant polyol est un polyéther-, un polyester- ou un polyéther-polyester-co-copolyester bifonctionnel contenant des fonctions OH primaires.

**13.** Formulation de photopolymère selon l'une quelconque des revendications 10 à 12, **caractérisée en ce que** le composant polyisocyanate est un polyisocyanate aliphatique ou un prépolymère contenant des groupes NCO primaires.

**14.** Formulation de photopolymère selon l'une quelconque des revendications 10 à 13, **caractérisée en ce que** le photoinitiateur est constitué d'une combinaison de colorants dont les spectres d'absorption couvrent au moins partiellement la plage spectrale allant de 400 à 800 nm, avec au moins un co-initiateur approprié.

**15.** Formulation de photopolymère selon l'une quelconque des revendications 10 à 14, **caractérisée en ce qu'**elle comprend en outre des plastifiants, de préférence des plastifiants de formule générale (II)

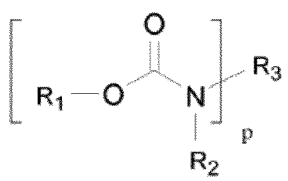

(II)

dans laquelle $p \geq 1$ et $\leq 8$ et $R^1$, $R^2$, $R^3$ représentent l'hydrogène et/ou indépendamment les uns des autres des radicaux organiques linéaires, ramifiés, cycliques et/ou hétérocycliques non substitués ou éventuellement également substitués avec des hétéroatomes, au moins un des radicaux $R^1$, $R^2$, $R^3$ étant de préférence substitué avec au moins un atome de fluor, et $R^1$ étant de manière particulièrement préférée un radical organique contenant au moins un atome de fluor.

**16.** Utilisation d'une formulation de photopolymère selon l'une quelconque des revendications 10 à 15 pour la fabrication de matériaux holographiques, notamment pour l'enregistrement d'hologrammes en ligne, désaxés, à ouverture totale, à transfert, en lumière blanche, en transmission, de Denisyuk, désaxés en réflexion ou à bords éclairés, ainsi que de stéréogrammes holographiques.

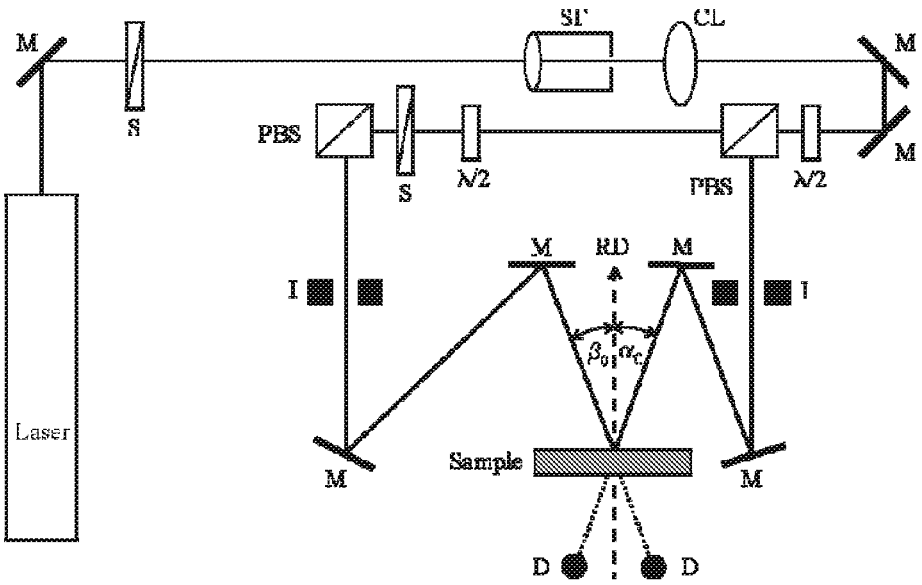


Figur1

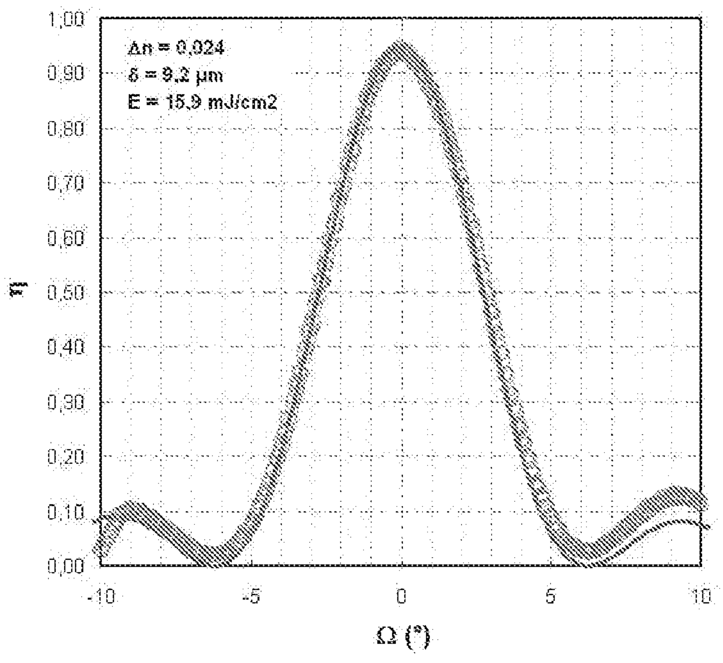


Figur 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008125229 A1
- WO 2008125199 A
- EP 09013770 A
- JP 2008247755 A
- EP 1627867 A1
- EP 0223587 A
- US 20100036013 A1


### In der Beschreibung aufgeführte Nicht-Patentliteratur


- **P. HARIHARAN.** Optical Holography. Cambridge University Press, 1996
- **HOUBEN-WEYL.** Methoden der organischen Chemie. Georg Thieme Verlag, 1961, vol. XIV/1, 433ff
- **CUNNINGHAM et al.** *RadTech'98 North America UV/EB Conference Proceedings,* 19. April 1998
- **KUTAL et al.** *Macromolecules,* 1991, vol. 24, 6872
- **YAMAGUCHI et al.** *Macromolecules,* 2000, vol. 33, 1152
- **NECKERS et al.** *Macromolecules,* 2000, vol. 33, 7761
- **LI et al.** *Polymeric Materials Science and Engineering,* 2001, vol. 84, 139
- **DEKTAR et al.** *J. Org. Chem.,* 1990, vol. 55, 639
- *J. Org. Chem.,* 1991, vol. 56, 1838
- **CRIVELLO et al.** *Macromolecules,* 2000, vol. 33, 825
- **GU et al.** *Am. Chem. Soc. Polymer Preprints,* 2000, vol. 41 (2), 1266
- **HUA et al.** *Macromolecules,* 2001, vol. 34, 2488-2494
- Chemistry & Technology of UV & EB Formulations For Coatings. SITA Technology. Inks & Paints, 1991, vol. 3, 61-328
- **NG, WILLIE W. ; HONG, CHI-SHAIN ; YARIV, AMNON.** Holographic interference lithography for integrated optics. *IEEE Transactions on Electron Devices,* 1978, ISSN 0018-9383, 1193-1200
- **H. KOGELNIK.** *The Bell System Technical Journal,* November 1969, vol. 48 (9), 2909-2947